(19) Europäisches Patentamt
European Patent Office
Office européen des brevets

(11) EP 2 786 766 A1

(12) **EUROPEAN PATENT APPLICATION**

(43) Date of publication:
**08.10.2014 Bulletin 2014/41**

(21) Application number: **13162532.9**

(22) Date of filing: **05.04.2013**

(51) Int Cl.:
***A61K 47/48*** *(2006.01)*
*C07K 7/06* *(2006.01)*
*C07K 7/08* *(2006.01)*
*A61K 38/08* *(2006.01)*
*A61K 38/10* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Ufpeptides S.r.l.**
**44122 Ferrara (IT)**

(72) Inventors:
- **Guerrini, Remo**
  **44121 FERRARA (IT)**
- **Salvadori, Severo**
  **44121 FERRARA (IT)**
- **Calo', Girolamo**
  **44121 FERRARA (IT)**

(74) Representative: **Cattaneo, Elisabetta et al**
**Notarbartolo & Gervasi S.p.A**
**Corso di Porta Vittoria, 9**
**20122 Milano (IT)**

# (54) Supramolecular aggregates comprising maleimido cores

(57) The invention relates to a supramolecular aggregate of formula (I) wherein A is an active substance, n is an integer from 4 to 16 and X is a core having at least four binding amino group. In a preferred embodiment the maleimido-funzionalized core X is selected from PWT1, PWT2 and PWT3. The supramolecular aggregates can be used in the field of drugs, vaccines, as ligands for GPCR, i.e. agonists as well as antagonist, as antibiotics and as diagnostics eventually in complex with radionuclides.

Figure 8

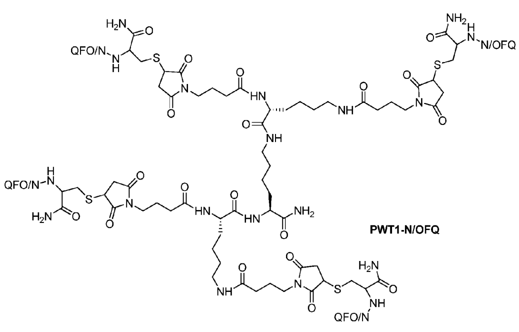

PWT1-N/OFQ

N/OFQ = Phe[1]-Gly-Gly-Phe-Thr-Gly-Ala-Arg[8]-Lys-Ser-Ala-Arg-Lys-Leu-Ala-Asn-Gln[17]

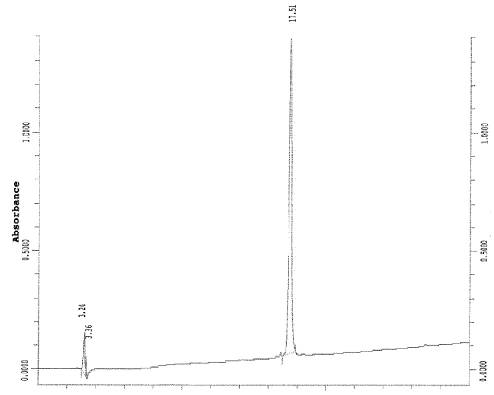


EP 2 786 766 A1

## Description

### Field of the invention

This invention relates to supramolecular aggregates of general formula (I) and their employment for targeting and optionally delivering active substances. In particular, the invention relates to new maleimido-functionalized cores forming the supramolecular aggregates with active substances.

### State of the art

Chemical strategies for the design of multimeric macrolecules have been described in the recent literature (B.D. Mather et al. Prog. Polym. Sci. 31 (2006) 487-531; K. Sadler et al. Reviews in Molecular Biotechnology 90, (2002) 195-229. The first example of such molecules of peptide nature was proposed by Tam and co-workers (K. J. Chang et al. Proc. Natl. Acad. Sci. USA 85, (1988) 4929-4933) as a solution to increase peptide size while maintaining the original amino acid sequence. Molecules designed by Tam were employed as "octopus immunogen" for generating antibody and this kind of immunization strategy has been named multiple antigen peptide (MAP) strategy. The observation of MAP resistance to proteolysis opened new perspectives for the use of peptides as drugs. To this regard, MAP strategy was applied for the synthesis of branched peptide as cobra-toxin antidotes, as antimicrobial agents, as carrier for tumor targeting etc (A. Pini et al. Current Protein and Peptide Science 9, (2008) 468-477). In addition, a small set of neuropeptides, including enkephalins, neurotensin and nociceptin/orphanin FQ (N/OFQ), were synthesized in monomeric and tetrabranched forms and incubated with human plasma and serum. All the tetrabranched neuropeptides retained full biological activity and generally showed higher stability to blood and brain protease activity (L. Bracci et al. J. Biol. Chem. 278, (2003) 56590-56595). Collectively, these data supported the development of branched peptide molecules as innovative therapeutics. However, the bigger are the final multimeric macromolecoles as supramolecular aggregates the more complex is their synthesis, purification and analytical characterization. In fact, the impurities generated during MAP assembly by standard solid phase synthesis techniques can not be removed during purification steps especially when long arms are needed. HPLC profile reported in figure 4 panel C of the paper (A. Pini et al. Current Protein and Peptide Science 9, (2008) 468-477) displays the low purity grade of the tetra-branched MAP investigated. The low purity grade of MAP molecules obtained by standard solid phase peptide synthesis techniques represents an important issue that need to be solved for the development of branched molecules, i.e.supramolecular aggregates as drugs.

The object of the present invention is therefore to provide supramolecular aggregate without using long and difficult purification steps and therefore to provide directly supramolecular aggregates in a purer form.

Furthermore, there is a need to provide new supramolecular aggragates, which are capable to link and target active substances in a stable and reproducible way.

Summary of the invention

The aforestated object has hence been attained by means of a supramolecular aggregate of formula I

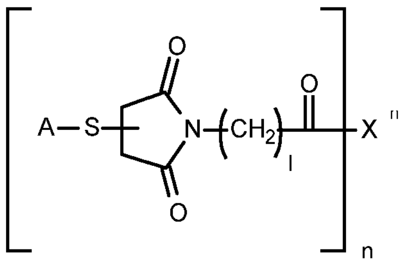

(I)

wherein

A-S- is an active substance derived with a thiol group or an active substance having a free thiol group
n is an integer from 4 to 16
X is a core having at least four binding amino groups
l is an integer from 1 to 10.
In a preferred embodiment said core X is selected from the group consisting of

(i) Formula (III)

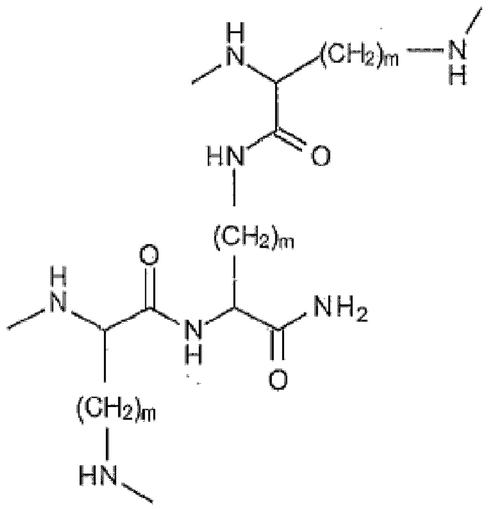

(III)

wherein m is an integer from 1 to 8
ii) Formula (IV)

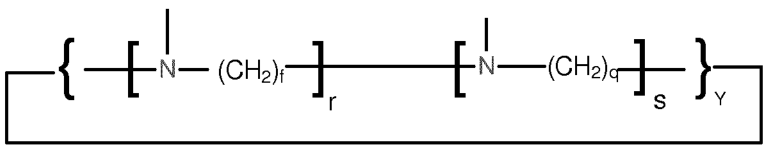

(IV)

wherein f, q are independently each other an integer from 1 to 8,
r and s are independently each other an integer from 1 to 4 and Y is an integer from 2 to 8,
iii) Formula (V)

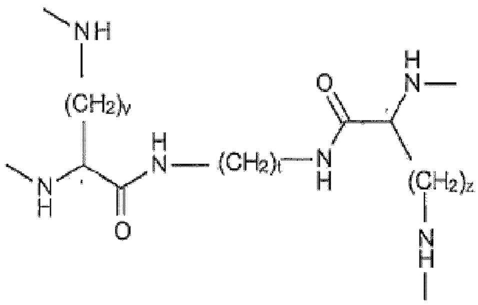

(V)

wherein v, t and z are independently each other from 1 to 8.
In the present invention when the definition "active substance" for A-S- is used, it is intended an active substance, such as a drug, a vaccine, a diagnostic substance to be targeted and delivered to the target site.

The cores of Formulas (III) and (IV) contain atom carbons which can have different stereochemical configurations. Therefore the present invention concerns cores X of Formulas (III) and (IV) which can be racemates, enantiomers and diastereoimers. Specifically, according to the invention, in Formulas (III) and (IV) the chiral centers are the following and are indicated through the symbol " * " placed on a carbon atom.

- Formula (III)

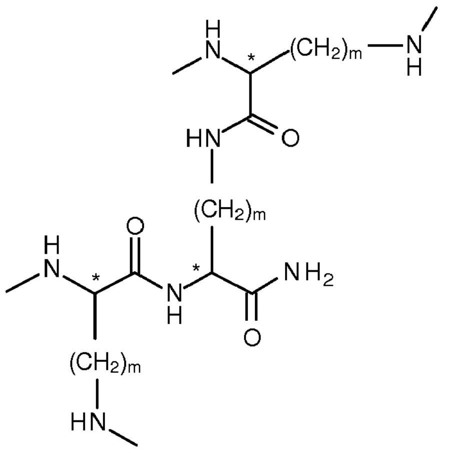

(III)

- Formula (V)

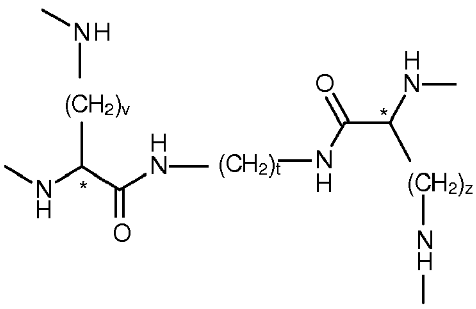

(V).

In another aspect, the present invention relates to a maleimido functionalized core of formula PWT1

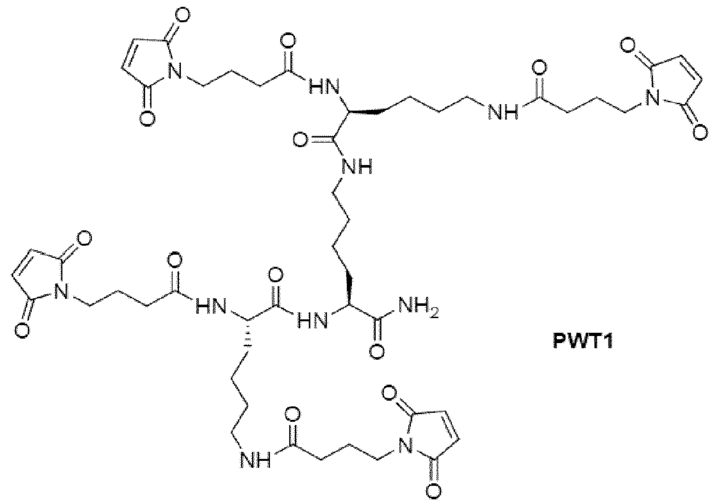

**PWT1**

In another aspect, the present invention relates to a maleimido functionalized core of formula PWT2

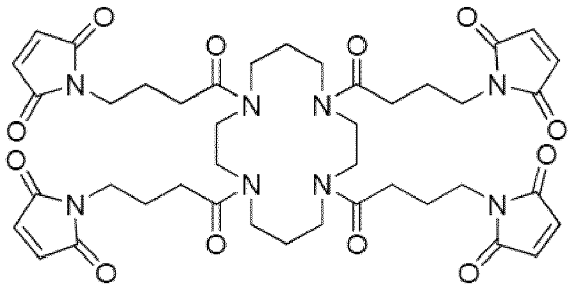

PWT2

In a still further aspect, the present invention relates to a maleimido functionalized core of formula PWT3.

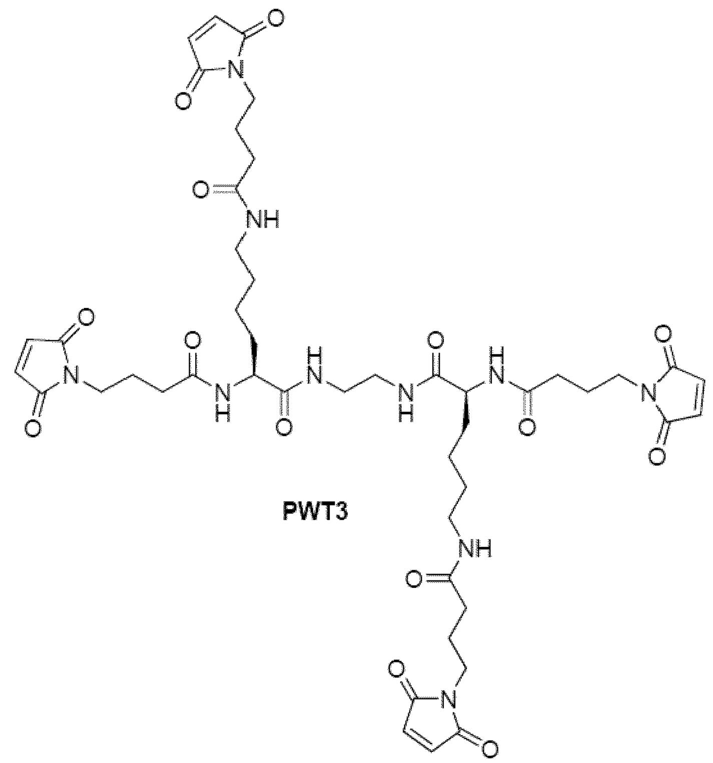

PWT3

The present invention therefore solves the above mentioned problem (i.e. low purity of branched molecules) by providing novel branched scaffolds as supramolecular aggregates containing a maleimido moiety. Such maleimido moieties linked to the branched core allow high yield thio-michael addition reaction and hence can be used for the chemoselective welding of molecules containing a thiol group. In view of the high yield and chemoselectivity of the reaction employed, the resulting supramolecular aggregates can be easily purified. In addition, the mild conditions required for and the quickness of the welding reaction between maleimido containing cores and thio-derived active substances extend advantageously the use of such approach to active substances with low chemical stability.

Furthermore, The new maleimido-functionalized cores of Formulas (III), (IV), and (V), more particularly PWT1, PWT2, and PWT3 resulted structurally capable to provide for not only a precise link at the thiol group with a specific active substance, but also to target such an active substance in a reproducible way to a targeted site.

In another aspect therefore the invention relates to a process for preparing the supramolecular aggregate of the invention comprising the step of reacting in a thiol-Michael addition n(A-SH) with a compound of Formula (II)

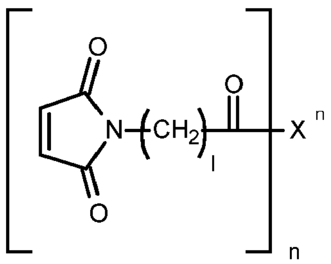

(II)

wherein
n is an integer from 4 to 16
X is a core having at least four binding amino groups
I is an integer from 1 to 10.

In another aspect the invention relates to a supramolecular aggregate for use in targeting, and optionally delivering, an active substance A to the target site.

The resulting supramolecular aggregates can be used for example, in the field of drugs, vaccines, as ligands for GPCR (agonists as well as antagonists), as antibiotics, as diagnostics (i.e. for PWT2 derivatives as radioligands) and in all the fields were the welding of a single bioactive pharmacophoric unit onto a multifunctional scaffold may be valuable.

**Description of the figures**

The invention is described in detail hereinafter with reference to the accompanying figures in which:

Figure 1 shows the synthesis scheme of PWT1
Figure 1A shows the synthesis scheme of PWT1-8
Figure 2 shows the synthesis scheme of PWT2
Figure 3 shows the synthesis scheme of PWT3
Figure 4 shows the mass spectrum of PWT1 (calculated M.W. 1062.48964)
Figure 4A shows the mass spectrum of PWT1-8 (calculated M.W. 2235.4051)
Figure 5 shows the mass spectrum of PWT2 (calculated M.W. 861.37775) (calculated PWT2 +Na MW 883.35969)
Figure 6 shows the mass spectrum of PWT3 (calculated M.W. 977.43632)
Figure 7 shows the analytical HPLC profile of the reaction mixture between PWT1 and [$Cys^{18}$]N/OFQ-$NH_2$
Figure 8 shows the chemical formula and HPLC profile of PWT1-N/OFQ
Figure 9 shows the chemical formula and HPLC profile of PWT2-N/OFQ
Figure 10 shows the chemical formula and HPLC profile of PWT3-N/OFQ
Figure 11 shows the mass spectrum of PWT1-N/OFQ (calculated M.W. 8706.91)
Figure 12 shows the mass spectrum of PWT2-N/OFQ (calculated M.W. 8505.69)
Figure 13 shows the mass spectrum of PWT3-N/OFQ (calculated M.W. 8621.80)
Figure 14 shows the mass spectrum of PWT1-NPS (calculated M.W. 10220.69)
Figure 15 shows the chemical formula and HPLC profile of PWT1-NPS

Figure 16. Calcium mobilization assay performed on CHONOP + $G\alpha_{qi5}$ cells. Concentration response curve to N/OFQ, PWT1-N/OFQ, PWT2-N/OFQ, and PWT3-N/OFQ (left panels). Inhibition response curve to SB-612111 against the stimulatory effect of N/OFQ and its PWT derivatives (right panels). Data are mean ± sem of at least 4 experiments made in duplicate.

Figure 17. Concentration response curve to N/OFQ and PWT1-N/OFQ (top panel), PWT2-N/OFQ (middle panel), and PWT3-N/OFQ (bottom panel) in the electrically stimulated mouse vas deferens. The values are means ± sem of 3 separate experiments.

Figure 18. Typical tracing of a concentration response curve to N/OFQ (top) and PWT1-N/OFQ (bottom). Note the slow kinetic of PWT1-N/OFQ action and lack of reversal after washing.

Figure 19. Concentration-response curve to N/OFQ (top left panel), PWT1-N/OFQ (top right panel), PWT2-N/OFQ (bottom left panel) and PWT3-N/OFQ (bottom right panel) obtained in the absence (control) and in the presence of SB-612111 (100 nM) in the electrically stimulated mouse vas deferens. The values are means ± sem of at least 3 separate experiments.

Figure 20. Concentration-response curve to N/OFQ (top left panel), PWT1-N/OFQ (top right panel), PWT2-N/OFQ (bottom left panel) and PWT3-N/OFQ (bottom right panel) obtained in vas deferens tissues taken from NOP(+/+)

and NOP(-/-) mice. The values are means ± sem of 3 separate experiments.

Figure 21. Mouse locomotor activity. Effects of equieffective i.c.v. doses of N/OFQ (10 nmol), PWT1-N/OFQ (0.25 nmol), PWT2-N/OFQ (0.25 nmol), and PWT3-N/OFQ (0.25 nmol) on animal distance travelled (top panels), immobility time (middle panels), and rearings (bottom panels). Results are displayed as time course from 4 PM to 9 AM (left panels) and as cumulative effects (right panels) Data are mean ± sem of 10 - 12 mice per group. *p < 0.05 vs saline according to ANOVA followed by the Bonferroni's test for multiple comparisons.

Figure 22. Calcium mobilization assay performed on HEK293rNPSR cells. Concentration response curve to NPS (neuropeptide S) and PWT1-NPS (top panel) and inhibition response curves to [$^t$Bu-D-Gly$^5$]NPS (middle panel) and SHA 68 (bottom panel). Data are mean ± sem of 3 separate experiments made in duplicate.

## Detailed description of the invention

The invention therefore relates to a supramolecular aggregate of formula I

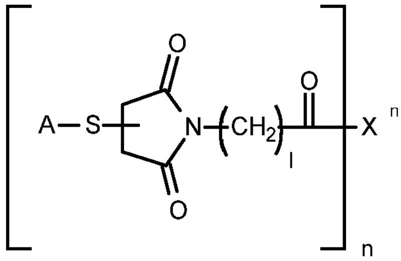

(I)

wherein

A-S- is an active substance derived with a thiol group or an active substance having a free thiol group

n is an integer from 4 to 16

X is a core having at least four binding amino groups

I is an integer from 1 to 10.

In a preferred embodiment said core X is selected from the group consisting of

(i) Formula (III)

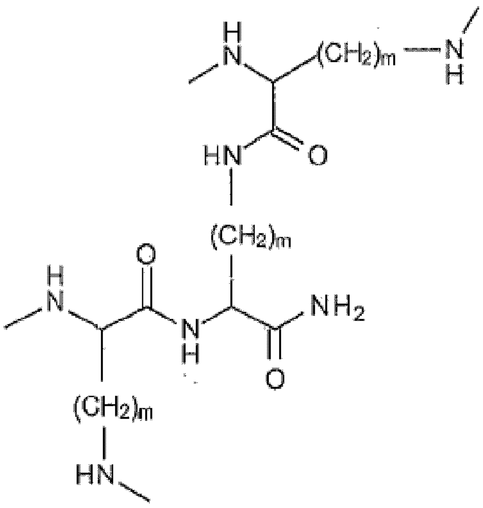

(III)

wherein m is an integer from 1 to 8

ii) Formula (IV)

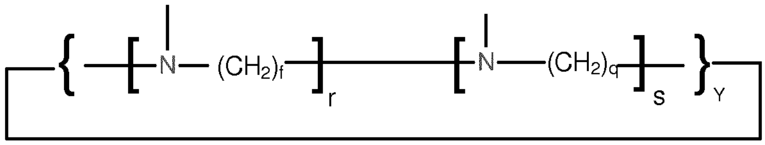

(IV)

wherein f, q are independently each other an integer from 1 to 8,
r and s are independently each other an integer from 1 to 4 and Y is an integer from 2 to 8,
iii) Formula (V)

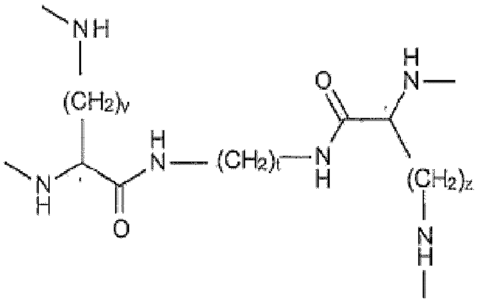

(V)

wherein v, t and z are independently each other from 1 to 8.

The cores of Formulas (III) and (IV) contain atom carbons which can have different stereochemical configurations. Therefore the present invention concerns cores X of Formulas (III) and (V) :

- Formula (III)

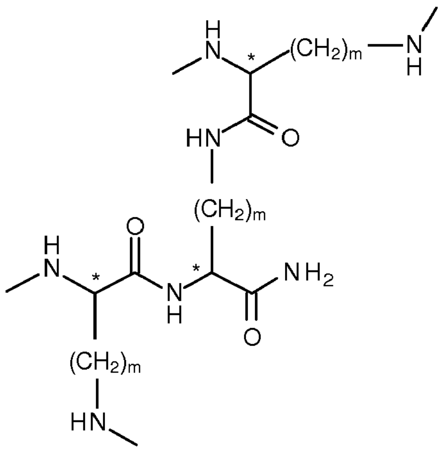

(III)

- Formula (V)

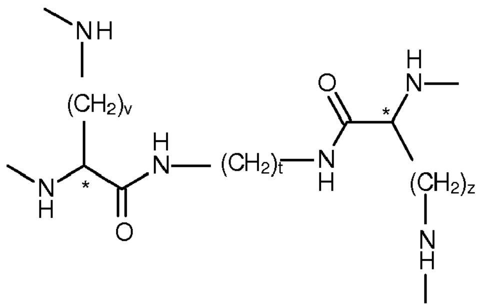

(V).

In a preferred embodiment therefore the core X is a racemate or in all the stereochemical configurations selected from enantiomers and diastereoisomers is

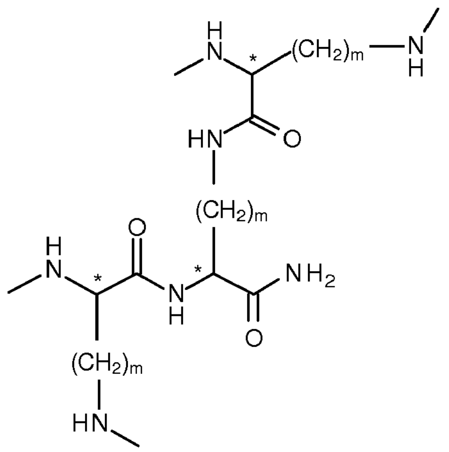

(III)

wherein m is an integer from 1 to 8 , preferably m is 4.

In a further preferred embodiment the core X is

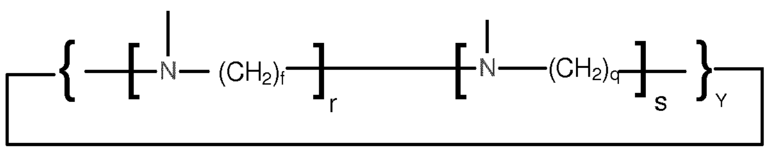

(IV)

wherein f, q are independently each other an integer from 1 to 8, preferably f is 3 and q is 2, r and s are independently each other an integer from 1 to 4, preferably they are equal to 1, and Y is an integer from 2 to 8, more preferably is 2.

In a still further preferred embodiment X in all the stereochemical configurations is

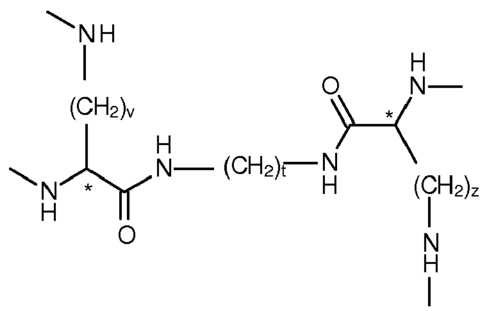

(V)

wherein v, t and z are independently an integer from 2 to 8, preferably v is 4, t is 2 and z is 4.

According to the invention in the supramolecular aggregate (I) X is linked to at least 4 maleimido fragments which are in turn linked through a thiol group to the active substance A, derived with a thiol group or having the thiol group.

The present invention relates hence to a maleimido-functionalized core of formula PWT1

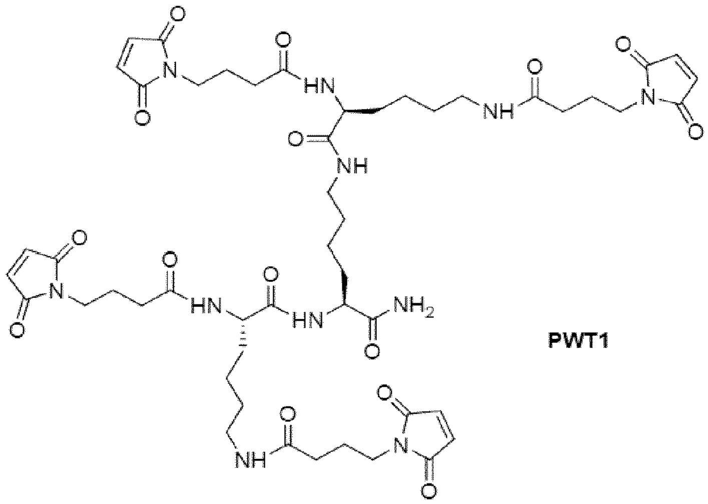

**PWT1**

In another aspect, the present invention relates to a maleimido-functionalized core of formula PWT2

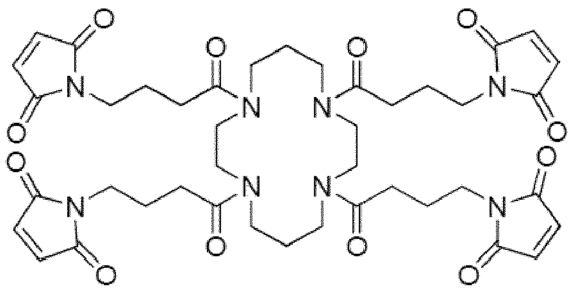

**PWT2**

In a still further aspect, the present invention relates to a maleimido-functionalized core of formula PWT3

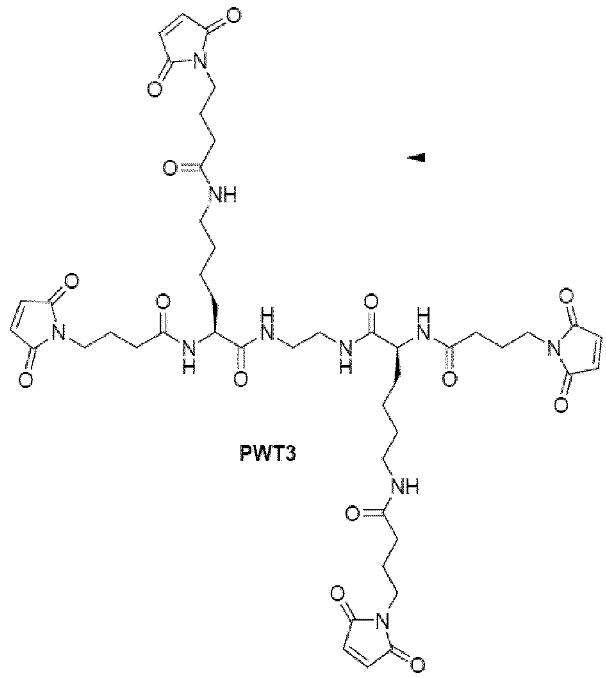

PWT3

The maleimido-functionalized core is linked to a thiol-active substance A.

In another aspect therefore the invention relates to a process for preparing the supramolecular aggregate of the invention comprising the step of reacting in a thiol-michael addition n(A-SH) with a compound of Formula (II)

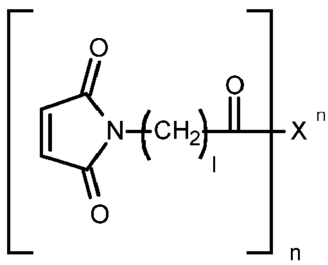

(II)

wherein

n is an integer from 4 to 16

X is a core having at least four binding amino groups

I is an integer from 1 to 10.

In a preferred embodiment the core X is selected from a compound of Formula (III), (IV) and (V).

In a more preferred embodiment the maleimido-functionalized X of formula (II) is PWT1, PWT2 or PWT3.

The active substance A-S- according to the invention can be any substance derived with a thiol group or containing a thiol group, which has to be targeted and optionally delivered to the target site.

The active substance A is preferably selected from the group consisting of

- Afamelanotide
  Ac-Ser-Tyr-Ser-Nle-Glu-His-D-Phe-Arg-Trp-Gly-Lys-Pro-Val-$NH_2$
  (a synthetic analog of the naturally-occurring melanocortin peptide hormone alpha-melanocyte stimulating hormone ($\alpha$-MSH))
- Aviptadil
  His-Ser-Asp-Ala-Val-Phe-Thr-Asp-Asn-Tyr-Thr-Arg-Leu-Arg-Lys-Gln-Met-Ala-Val-Lys-Lys-Tyr-Leu-Asn-Ser-Ile-Leu-Asn- $NH_2$
- Bivalirudin
  D-Phe-Pro-Arg-Pro-Gly-Gly-Gly-Gly-Asn-Gly-Asp-Phe-Glu-Glu-Ile-Pro-Glu-Glu-Tyr-Leu-OH
- Hirudin and related direct thrombin inhibitor peptides

- Bombesin
  Pyr-Gln-Arg-Leu-Gly-Asn-Gln-Trp-Ala-Val-Gly-His-Leu-Met-NH2
  (Pyr; pyroglutamic acid) and related analogues.
- Bradykinin
  Arg-Pro-Pro-Gly-Phe-Ser-Pro-Phe-Arg-OH, and related analogues
- Cetrorelix
  Ac-D-Nal-D-Cpa-D-Pal-Ser-Tyr-D-Cit-Leu-Arg-Pro-D-Ala-NH2 (Acetyl-D-3-(2'-naphtyl)-alanine-D-4-chlorophenylalanine-D-3-(3'-pyridyl)- alanine-L-serine-L-tyrosine-D-citrulline-L- leucine-L-arginine-L-proline-D- alanine-amide) and related gonadotropin-releasing hormone antagonist such as Abarelix, Degarelix, Ganirelix.
- Buserelin
  Pyr-His-Trp-Ser-Tyr-D-Ser(OtBu)-Leu-Arg-Pro-NHEt
  (Pyr; pyroglutamic acid) and related gonadotropin-releasing hormone agonist such as: Gonodorelin, Boserelin, Deslorelin, Histrelin, Leuprolide, Nafarelin, Triptorelin Goserelin.
- Carnosine
  Beta-Ala-His-OH
- Eledoisin
  Pyr-Pro-Ser-Lys-Asp-Ala-Phe-Ile-Gly-Leu-Met-NH2
  (Pyr; pyroglutamic acid)
- Enfuvirtide
  Ac-Tyr-Thr-Ser-Leu-Ile-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-Ser-Leu-Trp-Asn-Trp-Phe-$NH_2$
- Exenatide
  His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-$NH_2$
  and related glucagon-like-peptide-1 (GLP-1) receptor agonists such as: Liraglutide
- Ghrelin
  Gly-Ser-Ser(Octanoyl)-Phe-Leu-Ser-Pro-Glu-His-Gln-Arg-Val-Gln-Gln-Arg-Lys-Glu-Ser-Lys-Lys-Pro-Pro-Ala-Lys-Leu-Gln-Pro-Arg-OH
  and related growth hormone secretagogue receptor agonists such as: Sermorelin, Tesamorelin
- GHRP-6
  His-D-Trp-Ala-Trp-D-Phe-Lys-$NH_2$
  and related Growth hormone releasing peptide (GHRP) such as GHRP-2 (D-Ala-D-$\beta$-Nal-Ala-Trp-D-Phe-Lys-NH2) Hexarelin (His-2-Me-D-Trp-Ala-Trp-D-Phe-Lys-$NH_2$)
- Icatibant
  D-Arg-Arg-Pro-Hyp-Gly-Thi-Ser-Tic-Oic-Arg-OH
  and related bradikinin receptor (B2) antagonists
- Neuropeptide S
  Ser-Phe-Arg-Asn-Gly-Val-Gly-Thr-Gly-Met-Lys-Lys-Thr-Ser-Phe-Gln-Arg-Ala-Lys-Ser-OH
  and related analogues
- Opioid peptides, including enkephalin, dermorphin, deltorphin, dynorphin, endomorphin, nociceptin/orphanin FQ and related analogues
  Peptide sequences such as:

  Lys-Ala-Lys-Glu-Gly-Val
  Lys-Thr-Lys-Gln-Gly-Val
  Lys-Thr-Lys-Glu-Gly-Val
  Lys-Thr-Lys-Glu-Gln-Val
  Lys-Thr-Val-Glu-Gly-Ala
  Hexadecapeptide with all L and all D Ac-(Lys-Phe)$_8$

- Tetracosactide
  Ser-Tyr-Ser-Met-Glu-His-Phe-Arg-Trp-Gly-Lys-Pro-Val-Gly-Lys- Lys-Arg-Arg-Pro-Val-Lys-Val-Tyr-Pro-OH
  Synthetic ACTH analogues
- Thymopentin
  Arg-Lys-Asp-Val-Tyr-OH
- Thymosin $\alpha$-1
  Ac-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH

- Vx-001
  Tyr-Leu-Phe-Phe-Tyr-Arg-Lys-Ser-Val-OH
- Tuftsin
  Thr-Lys-Pro-Arg
- ACE inhibitors, including pseudo-peptides and peptidomimetics as alacepril, benazepril, captopril, cilazapril, delapril, enalapril, fosinopril, imidapril, lisinopril, moexipril, perindopril, quinapril, ramipril, spirapril, temocapril, trandolapril, zofenopril.
- H1 antagonists, includine ketotifen, azatadine, cetrizine, loratadine
- Beta2 adrenergic-agonists, including salbutamol, salmeterol, clenbuterol. Preferred active substances A are opioid peptides or neuropeptide S (NPS) correlated analogues NPS, more preferably said active substance is selected from the group consisting of enkephalin, dermorphin, deltorphin, dynorphin, endomorphin, nociceptin/orphanin FQ and NPS, still more preferably it is nociceptin/orphanin FQ or NPS.

Supramolecular aggregates are prepared by linking a maleimido-functionalized core X with a thiol-derived substance through the thiol-Michael addition reaction, preferably in the presence of an activating mixture.

The maleimido functionalized cores can be prepared both in solution and in solid phase organic chemistry synthesis, or any combination thereof. The maleimido functionalized core X (for example PWT1, PWT2, PWT3) are then reacted with an active substance containing or derived with a thiol group to give the supramolecular aggregates of the invention.

The new maleimido-functionalized cores of Formulas (III), (IV), and (V) resulted structurally capable to provide for not only a precise link at the thiol group with a specific active substance, but also to target such an active substance in a reproducible way to a target site. Without being bound to any theory the inventors deem that the surprising property of a precise, reproducible and stable link to an active substance and the efficacious targeting to a target site is due to the specific structure of maleimido-functionalized cores of Formulas (III), (IV) and (V).

In another aspect the invention relates to a supramolecular aggregate for use in targeting, and optionally delivering, an active substance A to the target site. Preferably the active substance is a drug.

The resulting supramolecular aggregates can be used, for example, in the field of vaccines, as ligands for GPCR (agonists as well as antagonists), as antibiotics, as diagnostics (eventually, for PWT2 in complex with a radionuclide) and in all the fields where the welding of a single molecule onto a multifunctional scaffold may be valuable.

In case of PWT2 the invention not only provide for a specific link to a thiol group of an active substance, but only provide for the complexation of the structure with other active substance. For example, for PWT2, the structure resulted capable to complex metals as radioisotopes.

The supramolecular aggregates of the invention do not affect the biological activity of the active substance A. Specifically the inventors showed that, in case of neuropeptides, the formation of the supramolecular aggregates does not affect the pharmacological activity (full agonism) of N/OFQ or NPS. In addition they showed that in vivo duration of action of PTW-N/OFQ derivatives is dramatically increased compared to that of N/OFQ. For exemplificative purposes general procedure to prepare PWT1, PWT2 and PWT3, their reaction with a thiol-containing or derived substance A are below reported.

<u>Synthesis of the maleimido cores (general procedure for the synthesis of PWT1, PWT2 and PWT3)</u>

In Figure 1 the synthesis of the maleimido core coined as PWT1 is reported. The commercially available Boc-Lys(Boc)-OH (1) was ammidate at the carboxylic function by using concentrated ammonia in the presence of 1-ethyl-3-(3-dimethylaminopropyl)carbodiimide hydrochloride/ethyl 2-cyano-2-(hydroxyimino)acetate (WSC/Oxp) as activating mixture. Compound 2 was treated with trifluoroacetic acid (TFA) and coupled with Boc-Lys(Boc)-OH to yield compound 4. Again, the treatment of 4 with TFA produced the tetra-amino core 5 that was acylated with the commercially available 4-(2,5-dioxo-2,5-dihydro-1 H-pyrrol-1-yl)butanoic acid (6). Tetra-maleimido functionalized core PWT1 was then obtained.

In Figure 2 the general procedure for preparing the maleimido core coined as PWT2 is reported. The commercially available compounds 6 and 7 were reacted in the presence of the activating mixture WSC/Oxp to give in one step the desired product PWT2 in high yield.

In Figure 3 the synthesis of the maleimido core coined as PWT3 is reported. The carboxylic function of compound 1 was activated in the presence of WSC/Oxp and reacted with ethylendiamine to give compound 8. The removal of the protecting group tert-butyloxycarbonyl (Boc) with TFA and the acylation of the tetra-amino core 9 with 6 generated the final product PWT3.

PWT1, PWT2 and PWT3 were then purified by preparative HPLC as explained below and their exact mass spectra are reported in Figures 4, 5, 6, respectively.

General procedure for preparative HPLC purification of PWT1, PWT2 and PWT3

Crude compounds PWT1, PWT2 and PWT3 as obtained as above were purified by preparative reversed-phase HPLC using a Water Delta Prep 4000 system with a Jupiter column C18 (250 x 30 mm, 300 A, 15 μm spherical particle size). The column was perfused at a flow rate of 20 mL/min with mobile phases A ($H_2O$ in 0.1 % TFA) and B (60% $CH_3CN$ in 0.1 % TFA) and the following gradient: t0 A90%; t25 A40%; t30 A20%; t35 A 90% was employed for the elution of compounds. The maleimido functionalized core X (PWT1, PWT2, PWT3) are then reacted with active substance containing or derived with a thiol group. The exemplificative general procedure to link the thiol-containing active substance A and the maleimido functionalized core X (PWT) follows.

General procedure of thio-Michael addition reaction between PWT cores and molecules containing a thiol group

To a stirred solution of PWT (mmol 0.00169) in $CH_3CN/H_2O$ (1 mL) a solution of thiol containing active substance A (mmol 0.00744, 10% excess) in $CH_3CN/H_2O$ (1 mL) was added followed by $NaHCO_3$ 5% (0.1 mL). The reaction was stirred at room temperature and monitored by analytical HPLC. PWT cores react completely and disappear from HPLC chromatogram in few minutes. Then the reaction mixture is purified directly by preparative HPLC to give the final product in high yield.

The examples relating to the detailed procedure for preparing PWT1, PWT2 and PWT3 and their reaction with thiol-containing substance A follow.

Example 1. Detailed procedure for the synthesis of PWT1 (reference to Figure 1)

Compound 2. To a stirred solution of compound 1 (1 mmol) in dimethylformamide (DMF) (5 mL) at 0 °C, WSC (1.1 mmol) and Oxp (1.1 mmol) were added. After 5 min a solution of $NH_3$ 30% (0.5 mL) was added and the reaction mixture stirred for 2 h. After evaporation of the solvent, the residue was treated with ethyl acetate (50 mL) and the organic layer extracted with 1 N HCl (3 x 15 mL), brine (2 x 15 mL) dried with $Na_2SO_4$ and evaporated to dryness.

Compound 3. Compound 2 (1 mmol) was treated with TFA (2 mL) for 0.5 h at room temperature. After this time, TFA was evaporated under vacuum to obtain a sticky oil.

Compound 4. To a stirred solution of compound 1 (1 mmol) in (DMF) (5 mL) at 0°C, WSC (1.1 mmol) and Oxp (1.1 mmol) were added. After 5 min a solution of compound 3 (0.5 mmol) in DMF (3 mL) deprotonated with N-methylmorpholine was added and the reaction mixture stirred for 4 h. After evaporation of the solvent, the residue was treated with ethyl acetate (50 mL) and the organic layer extracted with 1 N HCl (3 x 15 mL), $NaHCO_3$ 5% (3 x 15 mL), brine (2 x 15 mL) dried with $Na_2SO_4$ and evaporated to dryness. The residue was triturated with petroleum ether/diethyl ether; 1/1.

Compound 5. Compound 4 was treated with TFA as reported for compound 3.

PWT1. To a stirred solution of compound 6 (1.1 mmol) in (DMF) (5 mL) at 0 °C, WSC (1.2 mmol) and Oxp (1.2 mmol) were added. After 10 min a solution of compound 5 (0.25 mmol) in DMF (3 mL) deprotonated with N-methylmorpholine was added and the reaction mixture stirred for 4 h. After evaporation of the solvent, the residue was purified by preparative HPLC to give a white solid after lyophilization

Compound 5A. To a stirred solution of compound 1 (2 mmol) in (DMF) (8 mL) at 0°C, WSC (2.2 mmol) and Oxp (2.2 mmol) were added. After 5 min a solution of compound 5 (0.5 mmol) in DMF (5 mL) deprotonated with N-methylmorpholine was added and the reaction mixture stirred for 14 h. After evaporation of the solvent, the residue was washed with water ( 3 x 20 mL) and than evaporated to dryness. The residue was purified by flash chromatography using CH2C12/MeOH 9.5/0.5 as a solvent.

Compound 5Ab. Compound 5A was treated with TFA as reported for compound 3. PWT1-8. To a stirred solution of compound 6 (2.2 mmol) in (DMF) (10 mL) at 0 °C, WSC (2.4 mmol) and Oxp (2.4 mmol) were added. After 10 min a solution of compound 5Ab (0.25 mmol) in DMF (5 mL) deprotonated with N-methylmorpholine was added and the reaction mixture stirred for 24 h. After evaporation of the solvent, the residue was purified by preparative HPLC to give a white solid after lyophilization (Figure 1 A) with the expected MW (Figure 4A).

PWT1 and PWT1-8 con also be prepared by solid phase synthesis following the procedures reported in Benoiton, N. L. (2005) Chemistry of Peptide Synthesis, pp. 125-154, Taylor & Francis, Lond*on.* The molecule can be assembled starting from the C-terminal amide end toward the N-terminal end on an insoluble polymeric support. At the end of the synthesis the final PWT1 and PWT1-8 can be removed from the resin by hydrolysis with trifluoroacetic acid/water 95/5. After filtration of the exhausted resin, the solution can be evaporate to dryness and the residue purified by preparative HPLC .

Example 2. Detailed procedure for the synthesis of PWT2 (reference to Figure 2)

PWT2. To a stirred solution of compound 6 (1.1 mmol) in (DMF) (5 mL) at 0 °C, WSC (1.2 mmol) and Oxp (1.2 mmol) were added. After 10 min a solution of compound 7 (0.25 mmol) in DMF (3 mL) was added and the reaction mixture stirred for 4 h. After evaporation of the solvent, the residue was purified by preparative HPLC to give a highly hygroscopic white solid after lyophilization.

Example 3. Detailed procedure for the synthesis of PWT3 (reference to Figure 3)

Compound 8. To a stirred solution of compound 1 (1 mmol) in (DMF) (5 mL) at 0°C, WSC (1.1 mmol) and Oxp (1.1 mmol) were added. After 5 min ethylendiamine (0.5 mmol) was added and the reaction mixture stirred for 4 h. After evaporation of the solvent, the residue was treated with ethyl acetate (50 mL) and the organic layer extracted with 1 N HCl (3 x 15 mL), $NaHCO_3$ 5% (3 x 15 mL), brine (2 x 15 mL) dried with $Na_2SO_4$ and evaporated to dryness.

Compound 9. Compound 8 was treated with TFA as reported for compound 3.

PWT3. To a stirred solution of compound 6 (1.1 mmol) in (DMF) (5 mL) at 0 °C, WSC (1.2 mmol) and Oxp (1.2 mmol) were added. After 10 min a solution of compound 9 (0.25 mmol) in DMF (3 mL) deprotonated with N-methylmorpholine was added and the reaction mixture stirred for 4 h. After evaporation of the solvent, the residue was purified by preparative HPLC to give a white solid after lyophilization.

Example 4. Detailed procedure for preparing supramolecular aggregate of PWT1, PWT2 and PWT3 with nociceptin/orphanin FQ(N/OFQ-$NH_2$)

To a stirred solution of PWT1 (mmol 0.00169, MW 1062, mg 1.8) in $CH_3CN/H_2O$ (1 mL) a solution of [Cys18] N/OFQ-NH2 x 5TFA (mmol 0.00744, MW 2482, mg 18.5) in $CH_3CN/H_2O$ (1 mL) was added followed by $NaHCO_3$ 5% (0.1 mL). The reaction was monitored by analytical HPLC and appear to be completed in 4 min (Figure 7). The reaction mixture was purified by preparative HPLC to give the desired product (PWT1-N/OFQ) in quantitative yield.

The same reaction condition with the same stoichiometry was applied using PWT2 or PWT3 cores to give the final products (PWT2-N/OFQ and (PWT3-N/OFQ) in quantitative yield.

Chemical formula and HPLC profile of PWT1-N/OFQ; PWT2-N/OFQ and PWT3-N/OFQ are reported in figure 8, 9 and 10 respectively. Their mass spectra are reported in figure 11, 12 and 13

In addition, PWT1 core was reacted with [Cys21]NPS-NH2 using the same reaction condition and the same stoichiometry applied for PWT1-N/OFQ. Also in this case the reaction proceeded in few minutes and the final yield was quantitative. Chemical formula together with HPLC profile and mass spectrum of PWT1-NPS are reported in figure 14 and 15.

The high chemoselectivity and strength of the thio-Michael reaction together with the use of 10% excess of A-SH respect cores allow the complete consumption of the maleimido moiety independently from both the core shape (linear-branched as in PWT1 and PWT3 or cyclic-branched as in PWT2) and the peptide length.

The different chemical-physical properties of the final supramolecular aggregate respect the A-SH monomer allow an easy chromatographic purification of the reaction mixture with the possibility to recover not only the 100% of the desired final product but also the 10% excess of A-SH employed to bust to completion the reaction.

The above prepared supramolecular aggregates were tested for biological activity.

Example 5.

Biological activity of the supramolecular aggregates of the invention (PWT1, PWT2 and PWT3derivates of N/OFQ)

The aim of the present study was to pharmacologically characterize the *in vitro* and *in vivo* actions of a series of PWT derivatives of the nociceptin/orphanin FQ (N/OFQ) peptide. To this aim in vitro experiments were performed in vitro using cells expressing the N/OFQ peptide (NOP) as well as classical opioid (the MOP, DOP, and KOP) human recombinant receptors and the electrically stimulated mouse vas deferens, a N/OFQ sensitive pharmacological preparation. Moreover in vivo studies were performed investigating the effects of N/OFQ and PWT1-N/OFQ on mouse locomotor activity.

*Material and Methods*

*Calcium mobilization* - CHO cells stably co-expressing the human NOP, or KOP, or MOP receptor and the C-

terminally modified $G\alpha_{qi5}$ and CHO cells expressing the DOP receptor and the $G\alpha_{qG66Di5}$ protein were generated as previously described (Camarda et al., Naunyn Schmiedebergs Arch Pharmacol. 379, (2009) 599-607; Camarda and Calo, Methods Mol Biol.937, (2013) 293-306). Cells were maintained in culture medium consisting of Dulbecco's MEM/HAM'S F-12 (50/50) supplemented with 10% foetal calf serum, penicillin (100 IU/ml), streptomycin (100 μg/ml), fungizone (2.5 μg/ml), geneticin (G418; 200 μg/ml) and hygromycin B (200 mg/ml). Cell cultures were kept at 37 °C in 5% CO2 humidified air. In all cases experimental cultures were free from selection agents (hygromycin B, G418). When confluence was reached (3-4 days), cells were sub-cultured as required using trypsin//EDTA and used for experimentation. Cells were seeded at a density of 50,000 cells/well into 96-well black, clear-bottom plates. After 24 hours incubation the cells were loaded with medium supplemented with 2.5 mM probenecid, 3 μM of the calcium sensitive fluorescent dye Fluo-4 AM and 0.01% pluronic acid, for 30 min at 37 °C. Afterwards the loading solution was aspirated and 100 μl/well of assay buffer: Hank's Balanced Salt Solution (HBSS) supplemented with 20 mM HEPES, 2.5 mM probenecid and 500 μM Brilliant Black (Aldrich) was added. Stock solutions (1 mM) of N/OFQ, DPDPE, dynorphin A, dermorphin, and PWT compounds (PWT1-N/OFQ, PWT2-N/OFQ and PWT3-N/OFQ as prepared in above examples 1, 2, and 3) were made in distilled water and stored at -20 °C. SB-612111 was solubilized (10 mM) in DMSO. Serial dilutions of ligands for experimental use were made in HBSS/HEPES (20 mM) buffer (containing 0.03% BSA fraction V). After placing both plates (cell culture and compound plate) into the FlexStation II (Molecular Device, Union City, CA 94587, US), fluorescence changes were measured. On-line additions were carried out in a volume of 50 μl / well. To facilitate drug diffusion into the wells in antagonist type experiments, the present studies were performed at 37 °C and three cycles of mixing (25 μl from each well moved up and down 3 times) were performed immediately after antagonist injection to the wells.

*Electrically stimulated mouse vas deferens* - Tissues were taken from CD1 (30-35 g, Harlan, Italy) or CD1/C57BL6/J-129 NOP(+/+) or NOP(-/-) male mice. The mouse vas deferens were prepared as previously described (Calo et al., Eur J Pharmacol.311, (1996) R3-5). Tissues were suspended in 5 ml organ baths containing heated Krebs solution (composition in mM: NaCl 118.5, KCl 4.7, KH2PO4 1.2, $NaHCO_3$ 25, glucose 10 and CaCl2 2.5) oxygenated with 95% O2 and 5% CO2. The bath temperature was set at 33 °C. Tissues were continuously stimulated through two platinum ring electrodes with supramaximal rectangular pulses of 1 ms duration and 0.05 Hz frequency. A resting tension of 0.3 g was applied to the vas deferens. The electrically evoked contractions (twitches) were measured isotonically with a strain gauge transducer (Basile 7006, UgoBasile s.r.l., Varese, Italy) and recorded with the PC based acquisition system Power Lab (ADInstrument, USA). Following an equilibration period of 60 min, the contractions induced by electrical field stimulation were stable. At this time, cumulative concentration-response curves to N/OFQ, PWT supramolecular aggregates (PWT1-N/OFQ, PWT2-N/OFQ, PWT3-N/OFQ)or DPDPE were performed (0.5 log unit steps). In some experiments concentration response curves to agonists were performed in the absence or presence of SB-612111 (15 min preincubation time). *Locomotor activity* - All experimental procedures adopted for in vivo studies complied with the standards of the European Communities Council directives (86/609/EEC) and national regulations (D.L. 116/92). Male CD-1 mice (30-38 g, Harlan, Italy) and CD1/C57BL6/J-129 NOP(+/+) or NOP(-/-) male mice were used. They were housed in Plexiglas® cages (Tecniplast, Italy), under standard conditions (22 °C, 55% humidity, 12 h light-dark cycle, lights on 7.00 am) with food and water ad libitum for at least 5 days before experiments began. Experiments were performed according to the procedures previously described (Guerrini et al., J Med Chem.52, (2009) 524-529). For these experiments the ANY-maze video tracking system was used (Ugo Basile, application version 4.52c Beta). Mice were positioned in a square plastic cage (40 × 40 cm), one mouse per cage. Four mice were monitored in parallel. Mouse horizontal activity was monitored by a camera while vertical activity was measured by an infrared beam array. Animals locomotion was recorded for 60 min. The parameters measured were cumulative distance travelled (total distance in m that the animal travelled during the test), immobility time (the animal is considered immobile when 90% of it remains in the same place for a minimum of 2.5 s), and the number of rearings (the number of beam breaks due to vertical movements). N/OFQ, PWT1-N/OFQ,PWT2-N/OFQ, and PWT3-N/OFQ were given intracerebroventricularly (i.c.v., 2 μl/mouse). Free hand i.c.v. injections were given, under light isofluorane anaesthesia (just sufficient to produce a loss of the righting reflex), in the left ventricle according to literature procedures (Laursen and Belknap, J Pharmacol Methods.16, (1986) 355-357.

*Data analysis and terminology*- In vitro data were expressed as mean ± sem of at least three separate experiments. In calcium mobilization experiments, maximum change in fluorescence, expressed as percent over the baseline fluorescence, was used to determine agonist response. Non-linear regression analysis using GraphPad Prism software (5.0) allowed logistic iterative fitting of the resultant responses and the calculation of agonist potencies and maximal effects. Agonists potencies were given as $pEC_{50}$ (the negative logarithm to base 10 of the molar concentration of an agonist that produces 50% of the maximal possible effect). SB-612111 antagonist properties were evaluated in inhibition response curve experiments vs. a fixed concentration of N/OFQ or PWT compounds approximately corresponding to its $EC_{80}$; the antagonist potency was expressed as $pK_B$ derived from the following equation:

$$pK_B = -\log(IC_{50}/([2 + ([A]/EC_{50})^n]^{1/n} - 1))$$

where $IC_{50}$ is the concentration of antagonist that produces 50% inhibition of the agonist response, [A] is the concentration of agonist, $EC_{50}$ is the concentration of agonist producing a 50% maximal response and n is the Hill coefficient of the concentration response curve to the agonist.

In tissues experiments the antagonist effect of SB-612111 has been assessed by performing concentration response curve to agonists in the absence and in the presence of a fixed concentration of antagonist. SB-612111 potency was derived from the Gaddum Schild equation:

$$pK_B = -\log((CR - 1)/[antagonist])$$

assuming a slope value equal to unity, where CR indicate the ratio between agonist potency in the presence and absence of antagonist.

In vivo data are expressed as mean ± sem of n animals. Data were analysed using one-way analysis of variance (ANOVA) followed by Dunnett post hoc test. Differences were considered statistically significant when $p < 0.05$.

*Results*

*Calcium mobilization assay* - In $CHO_{NOP}$ cells stably expressing the $G\alpha_{qi5}$ chimeric protein and human NOP recombinant receptor, N/OFQ and its PWT derivatives evoked a concentration dependent stimulation of calcium release (Figure 16, left panels). N/OFQ displayed high potency ($pEC_{50}$ 9.39) and maximal effects (237 ± 15% over the basal values). PWT derivatives of N/OFQ mimicked the peptide stimulatory effects showing similar maximal effects but slightly lower potency ($pEC_{50}$ 8.75, 8.83, and 9.16 for PWT1-N/OFQ, PWT2-N/OFQ and PWT3-N/OFQ, respectively). When tested in CHO cells expressing the $G\alpha_{qi5}$ protein but not the NOP receptor, N/OFQ and its PWT derivatives were found inactive up to 1 μM (data not shown). Inhibition response experiments were performed by testing increasing concentrations (10 pM - 10 μM) of the standard NOP antagonist SB-612111 against a fixed concentration of agonist approximately corresponding to its $EC_{80}$ (10 nM for N/OFQ and 30 nM for PWT1-N/OFQ, PWT2-N/OFQ, and PWT3-N/OFQ). SB-612111 concentration dependently inhibited the stimulatory effect of N/OFQ, displaying a $pK_B$ value of 8.01 in line with previous studies (8.16, Camarda et al., Naunyn Schmiedebergs Arch Pharmacol.379, (2009) 599-607). Similar results were obtained challenging the antagonist against the stimulatory effect of PWT1-N/OFQ, PWT2-N/OFQ and PWT3-N/OFQ (Figure 16, right panels). $pK_B$ values of SB-612111 vs PWT supramolecular aggregates were in the range 7.82 - 8.19 (Table 1).

Table 1. Calcium mobilization studies. Effects of N/OFQ and its PWT derivatives in CHO cells expressing the human NOP receptor and the $G\alpha_{qi5}$ chimeric protein.

| | $E_{max}$ | $pEC_{50}$ ($CL_{95\%}$) | SB-612111 $pK_B$ |
|---|---|---|---|
| N/OFQ | 237 ± 15 | 9.39 (9.23-9.57) | 8.01 (7.84-8.18) |
| $PWT_1$-N/OFQ | 235 ± 29 | 8.75 (8.35-9.15) | 7.82 (7.47-8.17) |
| $PWT_2$-N/OFQ | 239 ± 14 | 8.83 (8.47-9.18) | 8.23 (7.87-8.59) |
| $PWT_3$-N/OFQ | 233 ± 15 | 9.16 (9.08-9.24) | 8.19 (8.03-8.35) |

In order to assess the selectivity of action of PWT supramolecular aggregates, calcium mobilization experiments were also performed in cells expressing chimeric G protein and the classical opioid receptors MOP, DOP and KOP. In this series of experiments dermorphin, DPDPE and dynorphin A were used as standard agonists for MOP, DOP and KOP, respectively. In cells expressing the MOP receptor dermorphin evoked concentration dependent stimulatory effects with $pEC_{50}$ of 9.29 ($CL_{95\%}$ 9.19 - 9.38) and maximal effects of 135 ± 21 % (Table 2). The stimulatory effect of dermorphin were mimicked by dynorphin A that was however approx 300 fold less potent. In these cells DPDPE, N/OFQ and its PWT supramolecular aggregates, were found inactive up to 1 μM. In cells expressing the DOP receptor DPDPE evoked concentration dependent stimulatory effects with $pEC_{50}$ of 9.57 ($CL_{95\%}$ 9.03 - 10.11) and maximal effects of 86 ± 14 % (Table 2). Dynorphin A was also able to elicit calcium mobilization in these cells being however 100 fold less potent. All the other agonists were inactive up to 1 μM. Finally, in KOP cells dynorphin A stimulated calcium release in a concentration dependent manner with $pEC_{50}$ of 10.04 ($CL_{95\%}$ 9.93 - 10.16) and maximal effects of 225 ± 10 %. All other agonists were inactive in these cells. Collectively the results obtained in these experiments with standard ligands are in line with findings from literature (Reisine, Neuropharmacology34, (1995) 463-472). Results obtained with PWT supramolecular

aggregates demonstrated that the application of these chemical modifications do not affect the selectivity over classical opioid receptors displayed by the natural peptide N/OFQ.

Table 2. Calcium mobilization studies. Potencies of N/OFQ, its PWT derivatives, and standard opioid agonists in CHO cells expressing the human NOP or classical opioid receptors and chimeric proteins.

| | NOP | MOP | DOP | KOP |
|---|---|---|---|---|
| N/OFQ | 9.39 | inactive | inactive | inactive |
| $PWT_1$-N/OFQ | 8.75 | inactive | inactive | inactive |
| $PWT_2$-N/OFQ | 8.83 | inactive | inactive | inactive |
| $PWT_3$-N/OFQ | 9.16 | inactive | inactive | inactive |
| Dermorphin | inactive | 9.29 | inactive | inactive |
| DPDPE | inactive | inactive | 9.57 | inactive |
| Dynorphin A | inactive | 6.67 | 7.73 | 10.04 |

Inactive: inactive up to 1 μM.

*Electrically stimulated isolated tissue experiments* - In the isolated mouse vas deferens N/OFQ inhibited the twitch response to electrical field stimulation in a concentration dependent manner ($pEC_{50}$ 7.37 ($CL_{95\%}$ 7.29 -7.45), $E_{max}$ = 88 ± 1 % inhibition of control twitch) (Figure 17). PWT1-N/OFQ mimicked the inhibitory effect of N/OFQ producing similar maximal effects but showing approximately 3 fold higher potency ($pEC_{50}$ 7.85 ($CL_{95\%}$ 7.73 -7.97)). Similar results were obtained by testing PWT2-N/OFQ and PWT3-N/OFQ in the same preparation: both supramolecular aggregates elicited maximal effects similar to those of N/OFQ being more potent than the natural peptide (Figure 17). Interestingly enough, the inhibitory effect induced by N/OFQ takes place immediately after adding the peptide to the bath and was immediately reversible after washing the tissue (Figure 18). On the contrary, PWT1-N/OFQ induced a slow inhibitory effect which reaches the plateau only after 10 min. More importantly the effects induce by PWT1-N/OFQ were rather resistant to washing (Figure 18). Superimposable results both in terms of slow kinetic and washing resistant effects were obtained with PWT2-N/OFQ and PWT3-N/OFQ (not shown).

As shown in Figure 19, the effects of N/OFQ and of the three PWT supramolecular aggregates were evaluated in the electrically mouse vas deferent in the absence and presence of the NOP selective antagonist, SB-612111. SB-612111 100 nM did not modify per se the control twitches but produced a rightward shift of the concentration response curve to N/OFQ without modifying the maximal effect induced by the agonist (Figure 19, top left panel). A $pK_B$ value of 8.48 was derived from these data. This value of antagonist potency is superimposable to that previously reported in literature (8.50 (Spagnolo et al., J Pharmacol Exp Ther.321, (2007) 961-967). Similar findings were obtained by challenging SB-612111 versus PWT compounds. Of note the $pK_B$ value obtained for SB-612111 against PWT1-N/OFQ (8.02) was slightly lower than those obtained against N/OFQ or the other PWT compounds (Figure 19 and Table 3).

Table 3. Effects of N/OFQ and its PWT derivatives in the electrically stimulated mouse vas deferens.

| | $\mathbf{E_{max}}$ | $\mathbf{pEC_{50}}$ | **SB-612111 $\mathbf{pK_B}$** ($CL_{95\%}$) |
|---|---|---|---|
| N/OFQ | 88 ± 1 | 7.37 (7.29-7.45) | 8.48 (8.19-8.77) |
| $PWT_1$-N/OFQ | 89 ± 1 | 7.85 (7.73-7.97) | 8.02 (7.84-8.20) |
| PWT2-N/OFQ | 93 ± 2 | 7.78 (7.57-7.99) | 8.22 (7.61-8.83) |
| $PWT_3$-N/OFQ | 85 ± 2 | 8.08 (7.99-8.17) | 8.33 (8.17-8.49) |

The effects of N/OFQ and PWT supramolecular aggregates, and those elicited by the selective DOP receptor agonist DPDPE were investigated in the electrically stimulated mouse vas deferens taken from NOP(+/+) and NOP(-/-) mice. N/OFQ inhibited the electrically induced contractions in a concentration dependent manner with a potency value of 7.68 ($CL_{95\%}$ 7.53 - 7.83) and maximal effect of 92 ± 2% in tissues taken from NOP(+/+) mice being virtually inactive up to micromolar concentrations in those taken form NOP(-/-) animals (Figure 20 top left panel). On the contrary the selective DOP receptor agonist DPDPE produced similar inhibitory effects in NOP(+/+) and NOP(-/-) tissues (Table 4). These results confirmed previous findings (Fischetti et al., Peptides.30, (2009) 248-255). Under the same experimental conditions PWT supramolecular aggregates were assayed in tissues taken from NOP(+/+) and NOP(-/-) mice. In NOP(+/+) tissues, PWT1-N/OFQ mimicked the inhibitory effect of N/OFQ showing higher potency ($pEC_{50}$ 8.02 ($CL_{95\%}$ 7.83 -8.21)) and similar maximal effect (Emax = 91 ± 2%). Similar results were obtained with PWT2-N/OFQ and PWT3-N/OFQ (Figure 20). In NOP(-/-) tissues the three PWT compounds maintained the ability to inhibit the electrically induced contractions showing however reduced potency by more than 10 fold for PWT1-N/OFQ and more than 100 fold for PWT2-N/OFQ and PWT3-N/OFQ (Figure 20). The values of potency and maximal effects obtained with N/OFQ and

PWT supramolecular aggregates derivatives and with DPDPE have been summarized in Table 4.

Table 4. Effects of N/OFQ, PWT supramolecular aggregates, and DPDPE in mouse vas deferent tissues taken from NOP(+/+) and NOP(-/-) animals

| | **NOP(+/+)** | | **NOP(-/-)** | |
|---|---|---|---|---|
| | $pEC_{50}$ | $E_{max}$ | $pEC_{50}$ | Emax |
| N/OFQ | 7.68 (7.53 - 7.83) | 92 ± 2 | inactive | |
| $PWT_1$-N/OFQ | 8.02 (7.83 - 8.21) | 96 ± 1 | 6.72 (6.55 - 6.89) | 78 ± 6 |
| PWT2-N/OFQ | 7.92 (7.74 - 8.10) | 91 ± 2 | 5.85 (5.55 - 6.15) | ND |
| $PWT_3$-N/OFQ | 8.03 (7.93 - 8.13) | 95 ± 1 | 5.87 (5.65 - 6.09) | ND |
| DPDPE | 8.45 (8.19 - 8.71) | 91 ± 3 | 8.42 (8.16 - 8.68) | 94 ± 3 |

ND: maximal effect could not be determined because the concentration response curve was incomplete

*In vivo studies* - It is known that N/OFQ given supraspinally produces biphasic effects on locomotor activity in rodents: at high doses (nmol range) the peptide elicits a robust inhibitory effect while at low doses (pmol range) it may increase locomotion (for review see Calo et al., Br J Pharmacol.129, (2000) 1261-1283).In a series of experiments the acute effects of N/OFQ and PWT supramolecular aggregates given i.c.v. on spontaneous locomotor activity were evaluated in CD-1 mice. In these experiments mouse locomotor activity was recorded for 120 min. N/OFQ produced biphasic effects: at relatively low doses (pmole range) the peptide produced short lasting stimulatory effects while at higher doses (nmole range) it produced inhibitory effects. PWT1-N/OFQ, PWT2-N/OFQ and PWT3-N/OFQall mimicked the effects of the natural peptide being however about 40 fold more potent. In addition all PWT supramolecular aggregatesdisplayed slow onset of action and prolonged effects compared to the natural peptide. In order to investigate the different in vitro duration of action of N/OFQ compared to PWT supramolecular aggregates, the effects of equieffective doses of N/OFQ (10 nmole) and PWT1-N/OFQ, PWT2-N/OFQ and PWT3-N/OFQ(all at 0.25 nmole) were measured in a overnight experiment. In particular mice were injected i.c.v. at 11 AM and their locomotor activity was measured from 3 PM to 9 AM of the following day. As shown in Figure 21 mice injected with N/OFQ displayed a locomotor behaviour similar to that of saline injected animals. Thus the duration of action of the is less than 4 h. On the contrary animals injected with PWT1-N/OFQ displayed statistically significant reduced distance travelled and rearing behaviour associate with increased immobility time for the whole time course of the experiment. Very similar results were obtained with animals treated with PWT2-N/OFQ and PWT3-N/OFQ. Collectively these results demonstrated that supramolecular aggregates displayed higher potency in vivo associated with very long lasting effects.

Finally the in vivo selectivity of action of PWT2-N/OFQ has been investigated in knockout studies. NOP(+/+) and NOP(-/-) mice were injected with saline or PWT2-N/OFQ (0.25 nmole) and their locomotor activity assessed for 120 min post injecton. As summarized in Table X, PWT2-N/OFQ produced a statistically significant reduction of distance travelled and rearings in NOP(+/+) but not in NOP(-/-) mice.

Table 5. Effects of PWT2-N/OFQ on locomotor activity of NOP(+/+) and NOP(-/-) mice.

| | NOP (+/+) | | NOP (-/-) | |
|---|---|---|---|---|
| | Saline | PWT2-N/OFQ | Saline | PWT2-N/OFQ |
| Distance travelled (m) | 230 ± 26 | 104 ± 19* | 170 ± 10 | 203 ± 26 |
| Immobility time (s) | 1805 ± 378 | 3305 ± 390 | 2558 ± 454 | 2372 ± 525 |
| Rearings (n) | 911 ± 175 | 96 ± 19* | 663 ± 135 | 625 ± 181 |
| *p<0.05 vs saline, according to the Student t test for unpaired data. | | | | |

Collectively in vitro and in vivo studies demonstrated that supramolecular aggregates of N/OFQ behave as potent full agonists at human recombinant and native animal NOP receptors. The PWT modification has a slight effect on selectivity but this is associated with a huge impact on in vivo duration of action which is multiplied by several folds compared to that of the native sequence.

Example 6.

Biological activity of the supramolecular aggregates of the invention with NPS (PWT1-NPS)

*Materials and methods*

*Calcium mobilization assay* - Human Embryonic Kidney (HEK) 293 cells stably expressing the rat recombinant NPSR receptor (HEK293rNPSR) were maintained in DMEM medium supplemented with 10% fetal bovine serum, 2 mM, L-glutamine and Hygromycin (100 mg/L) and cultured at 37 °C in 5% CO2 humidified air. When confluence was reached (3-4 days), cells were sub-cultured as required using trypsin/EDTA and used for experimentation. HEK293rNPSR cells were seeded at a density of 50,000 cells/well into poly D-lysine coated, 96-well black, clear-bottom plates. After 24 hours incubation the cells were loaded with medium supplemented with 2.5 mM probenecid, 3 μM of the calcium sensitive fluorescent dye Fluo-4 AM and 0.01% pluronic acid, for 30 min at 37 °C. Afterwards the loading solution was aspirated and 100 μl/well of assay buffer: Hank's Balanced Salt Solution (HBSS) supplemented with 20 mM HEPES, 2.5 mM probenecid and 500 μM Brilliant Black (Aldrich) was added. Stock solutions (1 mM) of NPS, PWT1-NPS and [tBu-D-Gly$^5$]NPS were made in distilled water and stored at -20 °C. SHA 68 was solubilized (10 mM) in DMSO. Serial dilutions of ligands for experimental use were made in HBSS/HEPES (20 mM) buffer (containing 0.03% BSA fraction V). After placing both plates (cell culture and compound plate) into the FlexStation II (Molecular Device, Union City, CA 94587, US), fluorescence changes were measured. On-line additions were carried out in a volume of 50 μl/well. To facilitate drug diffusion into the wells in antagonist type experiments, the present studies were performed at 37 °C and three cycles of mixing (25 μl from each well moved up and down 3 times) were performed immediately after antagonist injection to the wells.

*Data analysis and terminology* - Data were expressed as mean ± sem of at least three independent experiments made in duplicate. In calcium mobilization experiments, maximum change in fluorescence, expressed as percent over the baseline fluorescence, was used to determine agonist response. Non-linear regression analysis using GraphPad Prism software (5.0) allowed logistic iterative fitting of the resultant responses and the calculation of agonist potencies and maximal effects. Agonists potencies were given as $pEC_{50}$ (the negative logarithm to base 10 of the molar concentration of an agonist that produces 50% of the maximal possible effect). SHA 68 and [tBu-D-Gly$^5$]NPS antagonist properties were evaluated in inhibition response curve experiments vs a fixed concentration of NPS and PWT1-NPS approximately corresponding to its $EC_{80}$; the antagonist potency was expressed as $pK_B$ derived from the following equation:

$$K_B = IC_{50}/([2 + ([A]/EC_{50})^n]^{1/n} - 1)$$

where $IC_{50}$ is the concentration of antagonist that produces 50% inhibition of the agonist response, [A] is the concentration of agonist, $EC_{50}$ is the concentration of agonist producing a 50% maximal response and n is the Hill coefficient of the concentration response curve to the agonist.

In vivo data are expressed as mean ± sem of n animals. Data were analysed using two-way analysis of variance (ANOVA; treatment x time) followed by Bonferroni's post hoc test. Differences were considered statistically significant when $p < 0.05$.

*Results*

Calcium mobilization assay - In $HEK293_{rNPSR}$ NPS evoked a concentration dependent stimulation of calcium release displaying high potency ($pEC_{50}$ 8.40) and maximal effects (283 ± 41 % over the basal values). These results are similar to those previously reported by us and others (see the review Guerrini et al., Med Res Rev. 30, (2010) 751-777). PWT1-NPS mimicked the stimulatory effect of the natural peptide showing similar maximal effects and slightly lower potency (Figure 22 top panel and Table 4).

Inhibition response experiments were performed by testing increasing concentrations (10 pM - 10 μM) of the standard NPSR antagonists SHA-68 and [$^t$Bu-D-Gly$^5$]NPS against fixed concentrations of NPS and PWT1-NPS approximately corresponding to their $EC_{80}$. SHA-68 and [$^t$Bu-D-Gly$^5$]NPS (Figure 22) inhibited in a concentration dependent manner the stimulatory effect of the two agonists with similar $pK_B$ values summarized in Table 6.

Table 6. Effects of the agonists NPS and PWT1-NPS, and of the antagonists SHA 68 and [$^t$Bu-D-Gly$^5$]NPS in calcium mobilization studies performed in HEK293rNPSR cells.

| | $E_{max}$ | $pEC_{50}$ | SHA 68 $pK_B$ | [$^t$Bu-D-Gly$^5$]NPS $pK_B$ |
|---|---|---|---|---|
| NPS | 286 ± 32 | 8.40 | 7.62 | 7.25 |
| $PWT_1$-NPS | 318 ± 24 | 7.64 | 7.75 | 6.90 |

Data are mean ± sem of 3 separate experiments made in duplicate.

Collectively in vitro studies demonstrated that PWT1-NPS behaves as a potent full agonist at the rat recombinant NPSR. Thus PWT chemical modifications can be applied at different peptide sequences (in these examples N/OFQ and NPS) without causing any major modification of their biological activity.

## Claims

**1.** A supramolecular aggregate of formula I

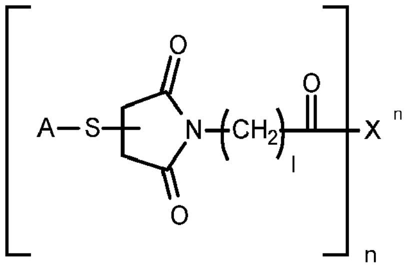

(I)

wherein
A-S- is an active substance derived with a thiol group or an active substance having a free thiol group
n is an integer from 4 to 16
X is a core having at least four binding amino groups
I is an integer from 1 to 10.

**2.** The supramolecular aggregate according to claim 1 wherein said core X is selected from the group consisting of

i) Formula (III)

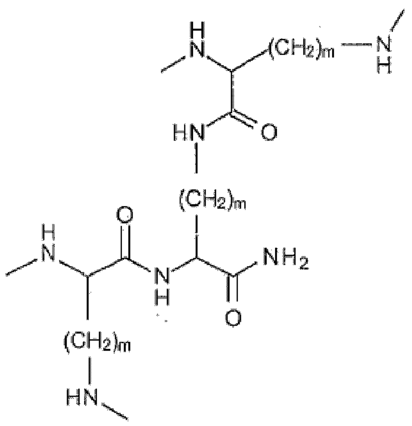

(III) or

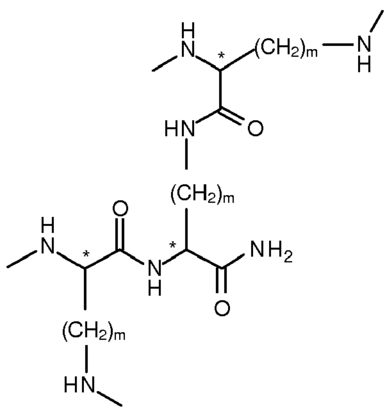

(III)

wherein m is an integer from 1 to 8 and the symbol "*" identifies chiral centers of a sterochemical configuration selected form enantiomers and diastereoisomers.

ii) Formula (IV)

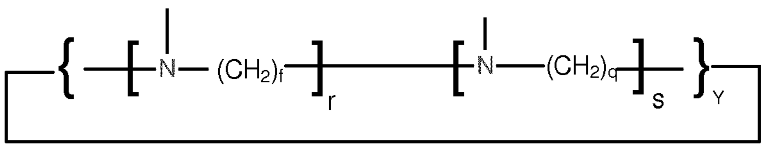

(IV)

wherein f, q are independently each other an integer from 1 to 8,
r and s are independently each other an integer from 1 to 4 and Y is an integer from 2 to 8, and
iii) Formula (V)

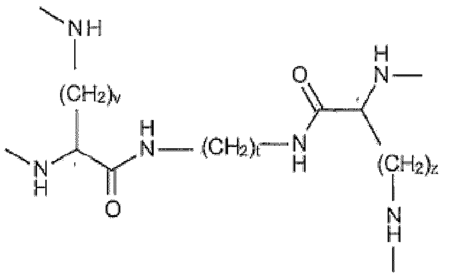

(V) or

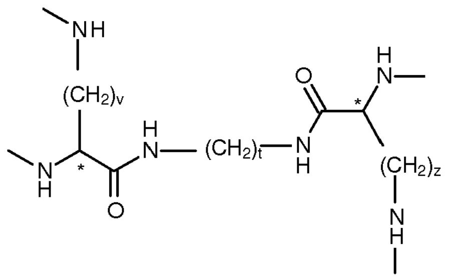

(V)

wherein v, t and z are independently each other from 1 to 8 and the symbol "*" identifies chiral centers of a sterochemical configuration selected form enantiomers and diastereoisomers.

**3.** The supramolecular aggregate according to claim 2, wherein when X is i) of Formula (III) and m is 4.

**4.** The supramolecular aggregate according to claim 2, wherein when X is ii) of Formula (IV), f is 3, q is 2, r and s are equal to 1, and Y is 2.

**5.** The supramolecular aggregate according to claim 2, wherein X is iii) of Formula (V) and v is 4, t is 2 and z is 4.

**6.** The supramolecular aggregate according to anyone of claims 1-5, wherein A [is an opioid peptide or neuropeptide S (NPS) or related analogues NPS, preferably said A is selected from the group consisting of enkephalin, dermorphin, deltorphin, dynorphin, endomorphin, nociceptin/orphanin FQ andneuropeptide S (NPS).

**7.** The supramolecular aggregate according to claim 6, wherein A is nociceptin/orphanin FQ or NPS.

**8.** A maleimido-functionalized core of formula PWT1

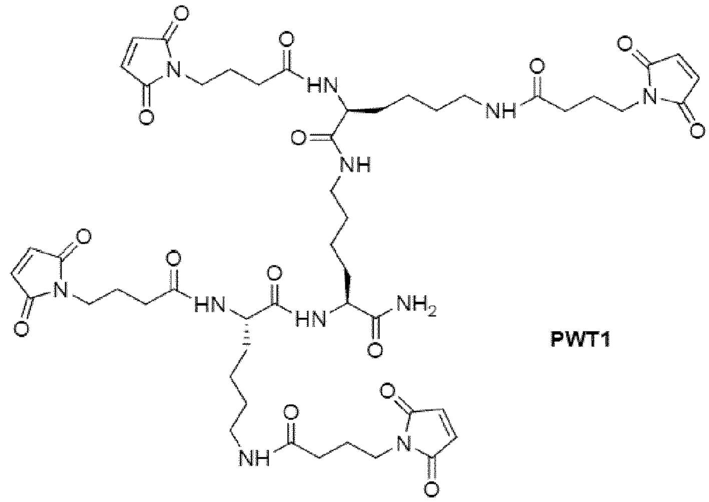

PWT1

**9.** A maleimido-functionalized core of formula PWT2

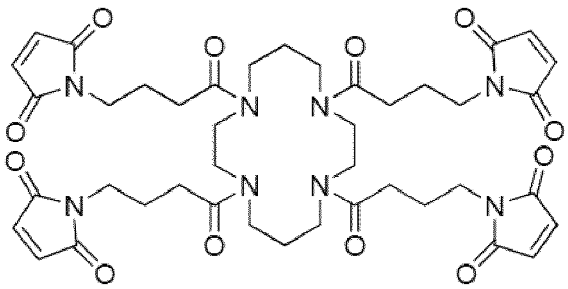

PWT2

**10.** A maleimido-functionalized core of formula PWT3

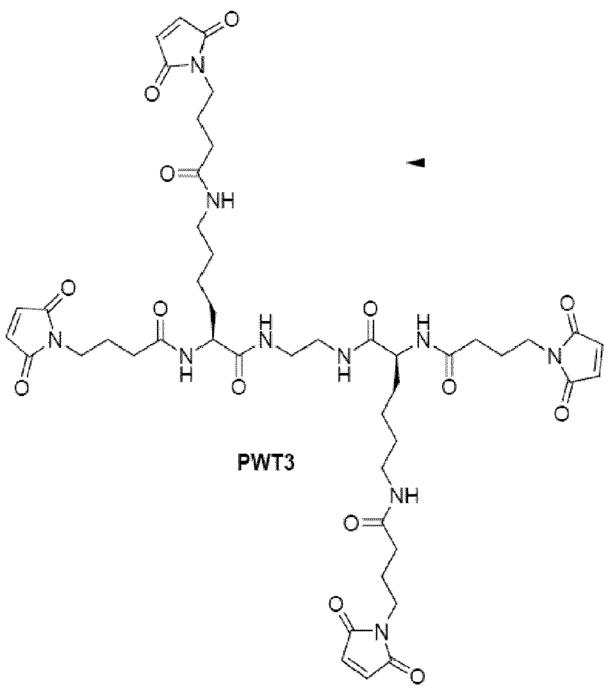

PWT3

**11.** A process for preparing the supramolecular aggregate of the invention of anyone of claims 1-7 comprising the step of reacting in a thiol-michael addition n(A-SH) with a compound of Formula (II)

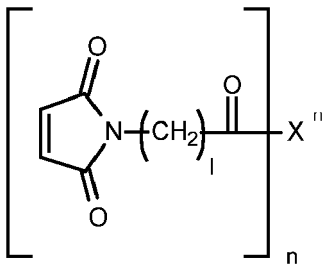

(II)

wherein
A is an active substance
n is an integer from 4 to 16
X is a core having at least four binding amino group.

**12.** The process according to claim 11, wherein the core X is selected from the group consisting of a compound of Formula (III), a compound of Formula (IV) and a compound of Formula (V).

**13.** The process according to claim 11, wherein the maleimido-functionalized X of formula (II) is PWT1, PWT2 or PWT3.

**14.** A supramolecular aggregate of Formula (I) of anyone of claims 1-7 for use in targeting, and optionally delivering, an active substance A to the target site.

**15.** The supramolecular aggregate of claim 14 wherein the active substance A is a drug, to be targeted to the suitable receptor site.

Figure 1

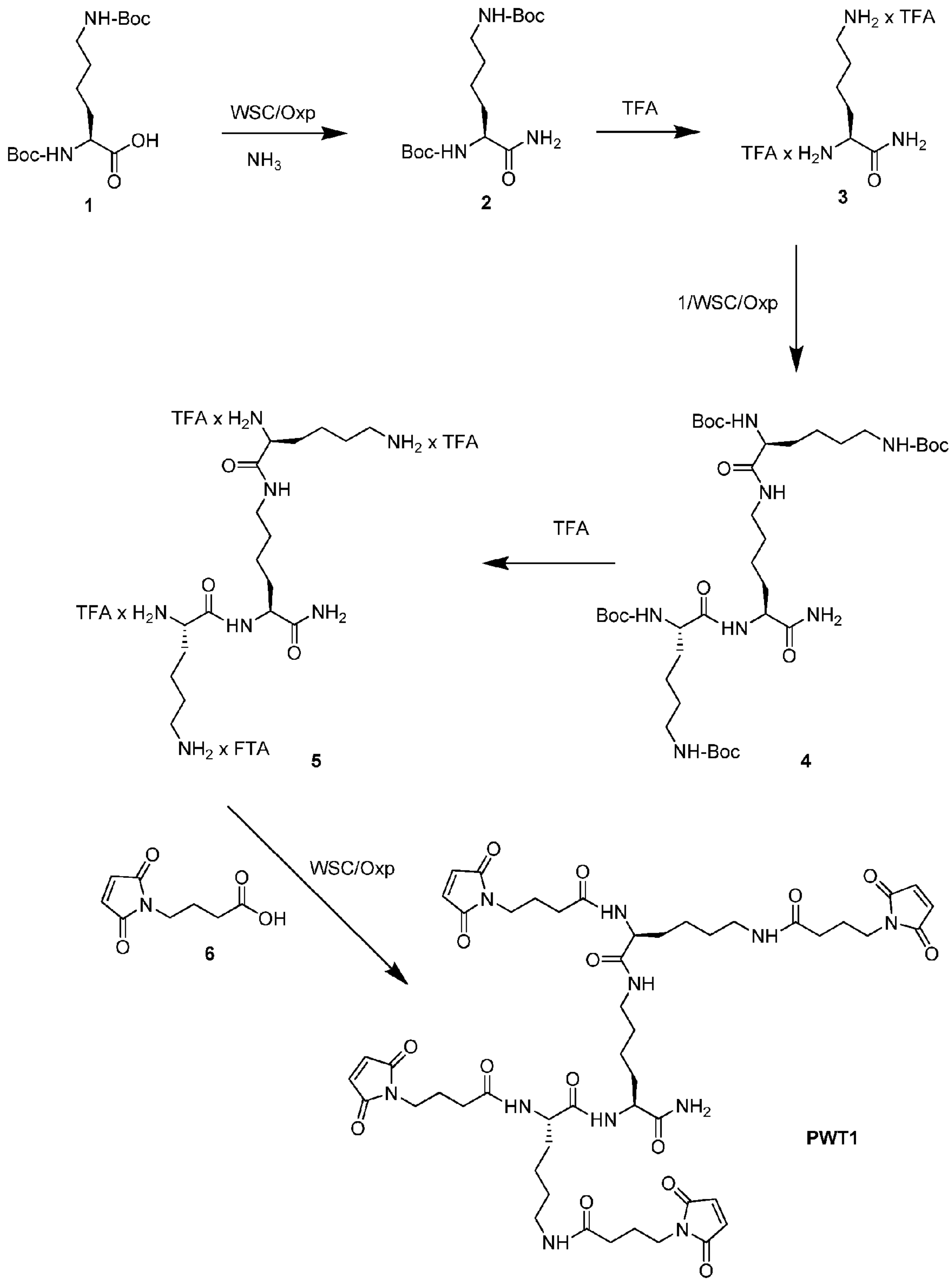


1 2 3 4 5 6 PWT1

Figure 1A

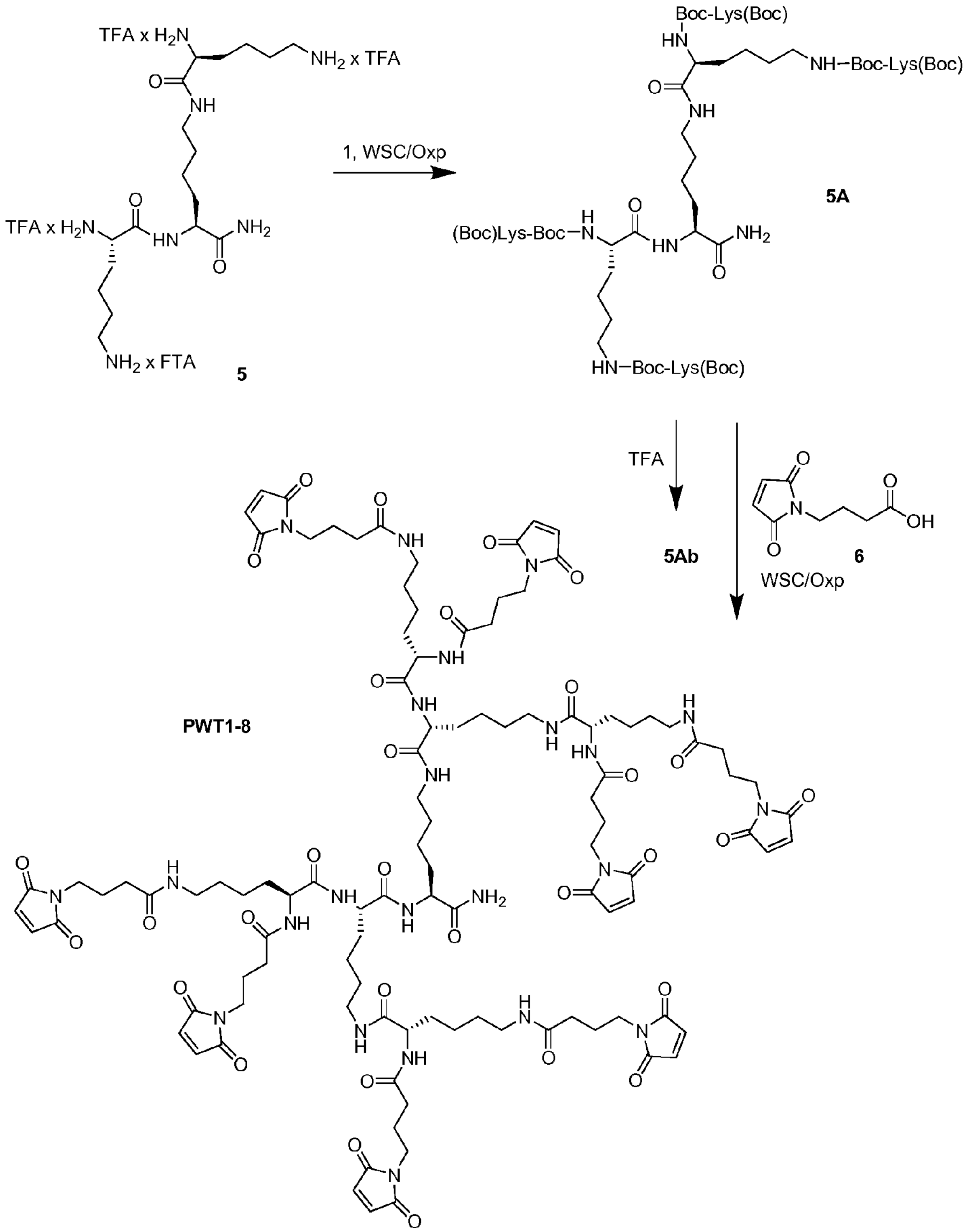


5

5A

5Ab

6

PWT1-8

Figure 2

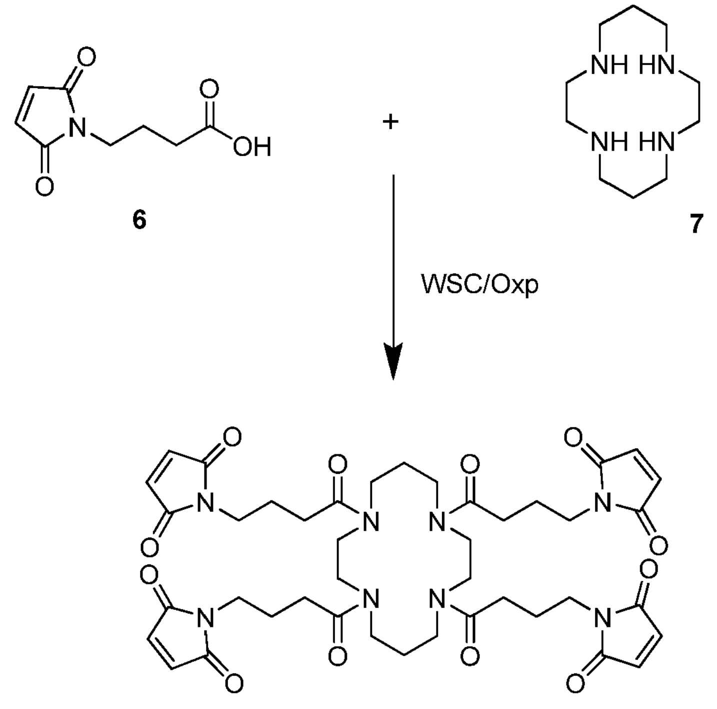


6

7

PWT2

Figure 3

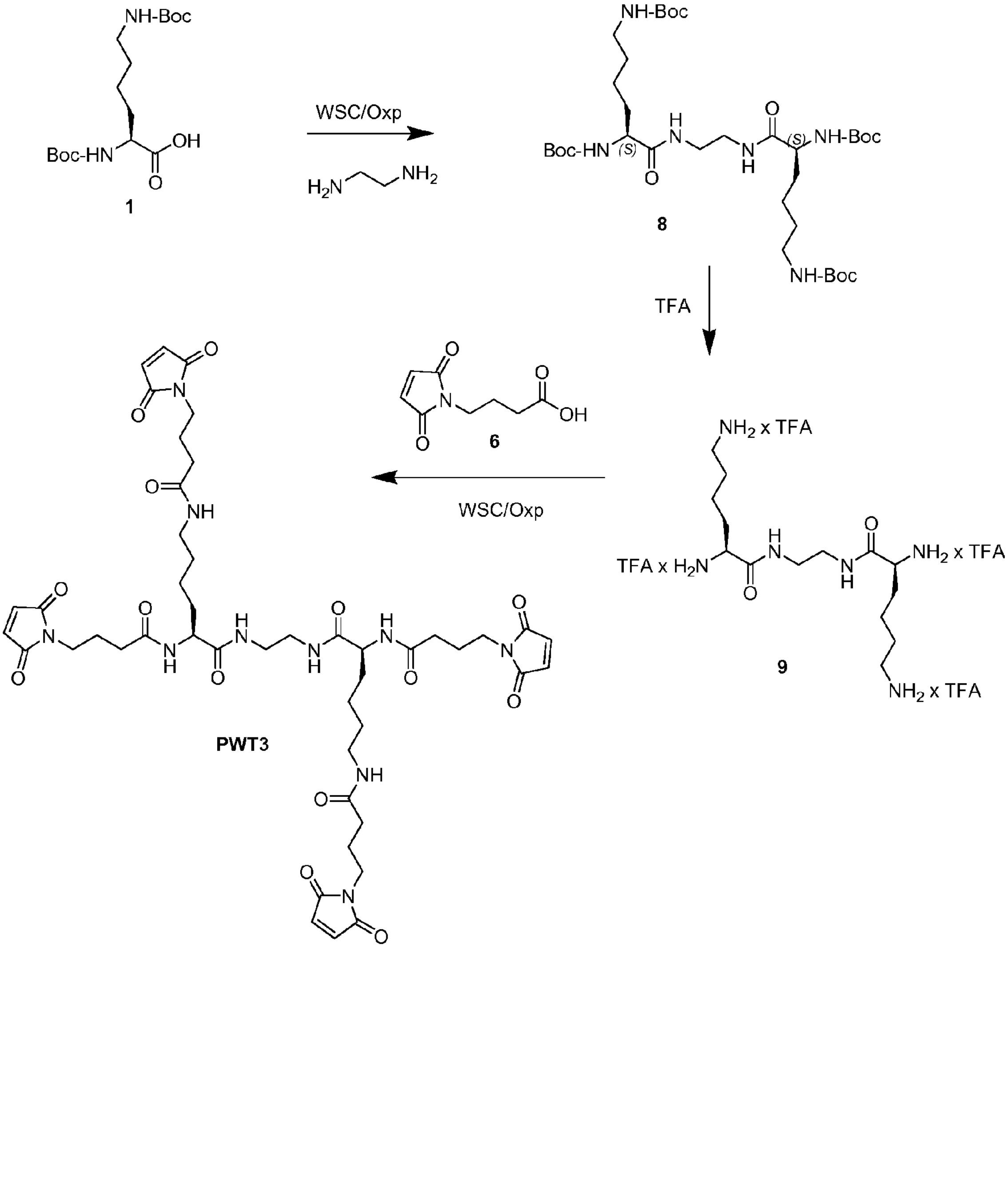

Figure 4

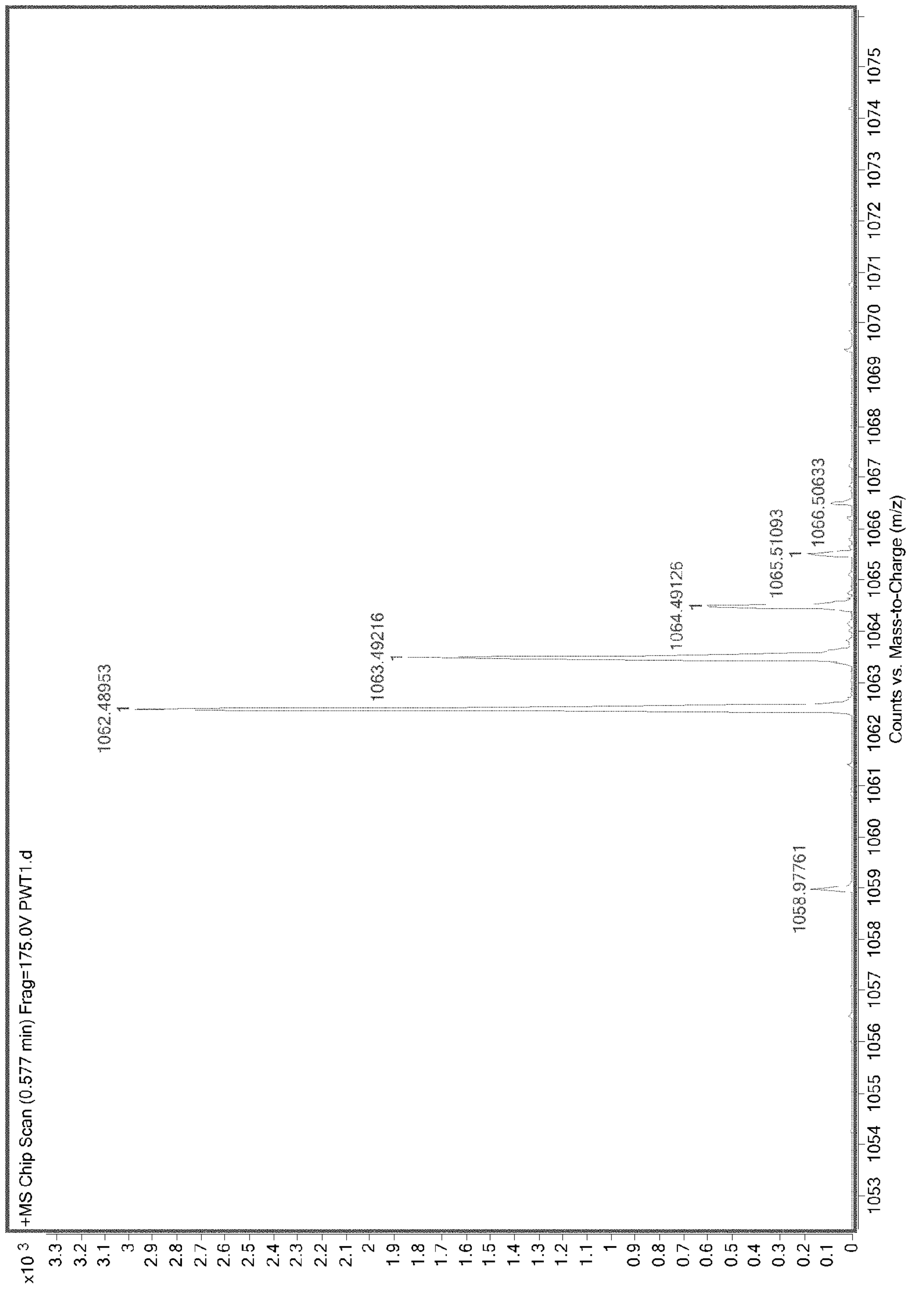
+MS Chip Scan (0.577 min) Frag=175.0V PWT1.d
x10 3
1062.48953
1063.49216
1064.49126
1065.51093
1066.50633
1058.97761
Counts vs. Mass-to-Charge (m/z)

Figure 4A

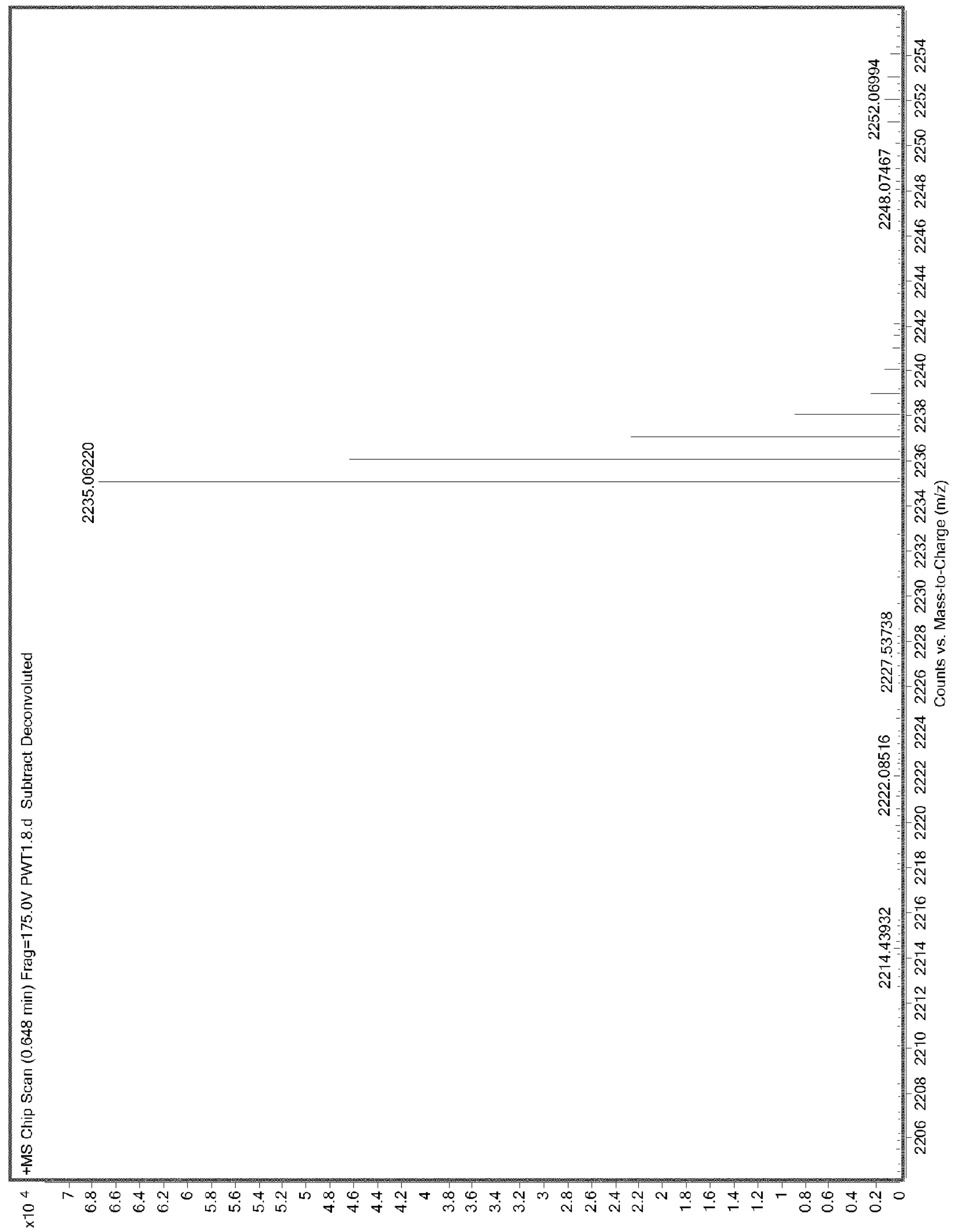

+MS Chip Scan (0.648 min) Frag=175.0V PWT1.8.d  Subtract Deconvoluted
x10 4
2235.06220
2214.43932
2222.08516
2227.53738
2248.07467
2252.06994
Counts vs. Mass-to-Charge (m/z)

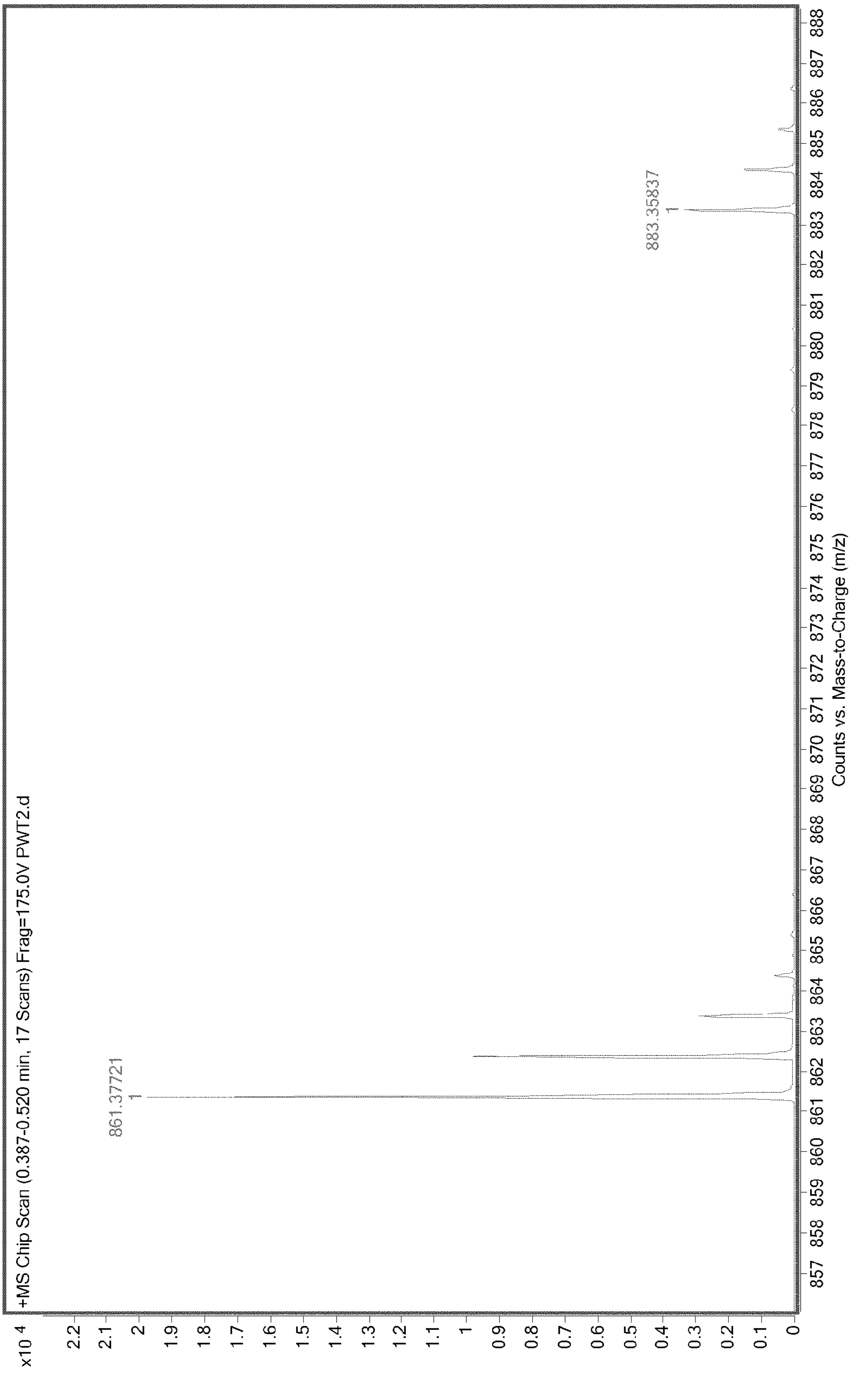
x10 4
+MS Chip Scan (0.387-0.520 min, 17 Scans) Frag=175.0V PWT2.d
861.37721
883.35837
Counts vs. Mass-to-Charge (m/z)

Figure 5

Figure 6

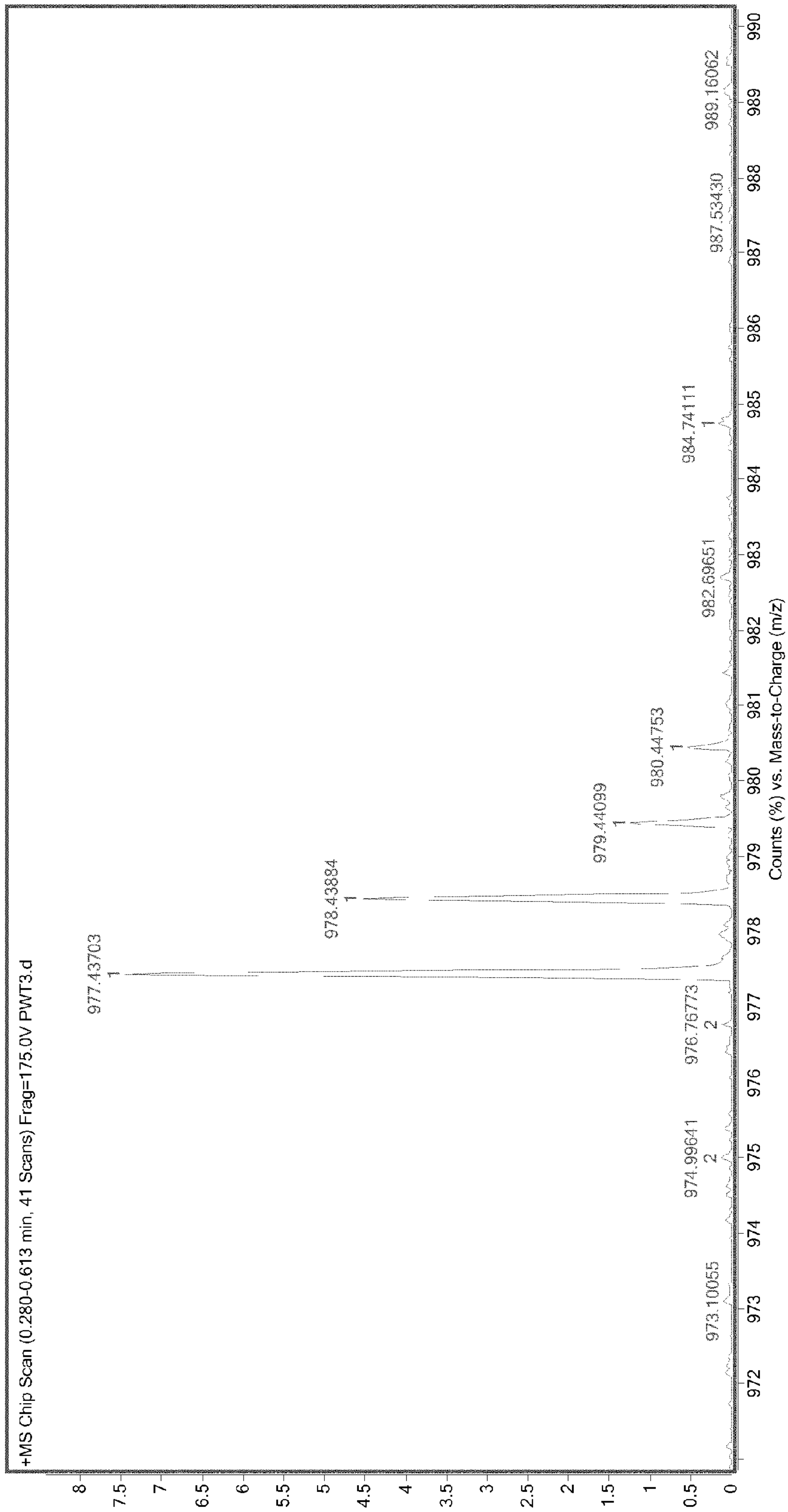
+MS Chip Scan (0.280-0.613 min, 41 Scans) Frag=175.0V PWT3.d
977.43703
1
978.43884
1
979.44099
1
980.44753
1
976.76773
2
974.99641
2
973.10055
982.69651
984.74111
1
987.53430
989.16062
Counts (%) vs. Mass-to-Charge (m/z)

Figure 7

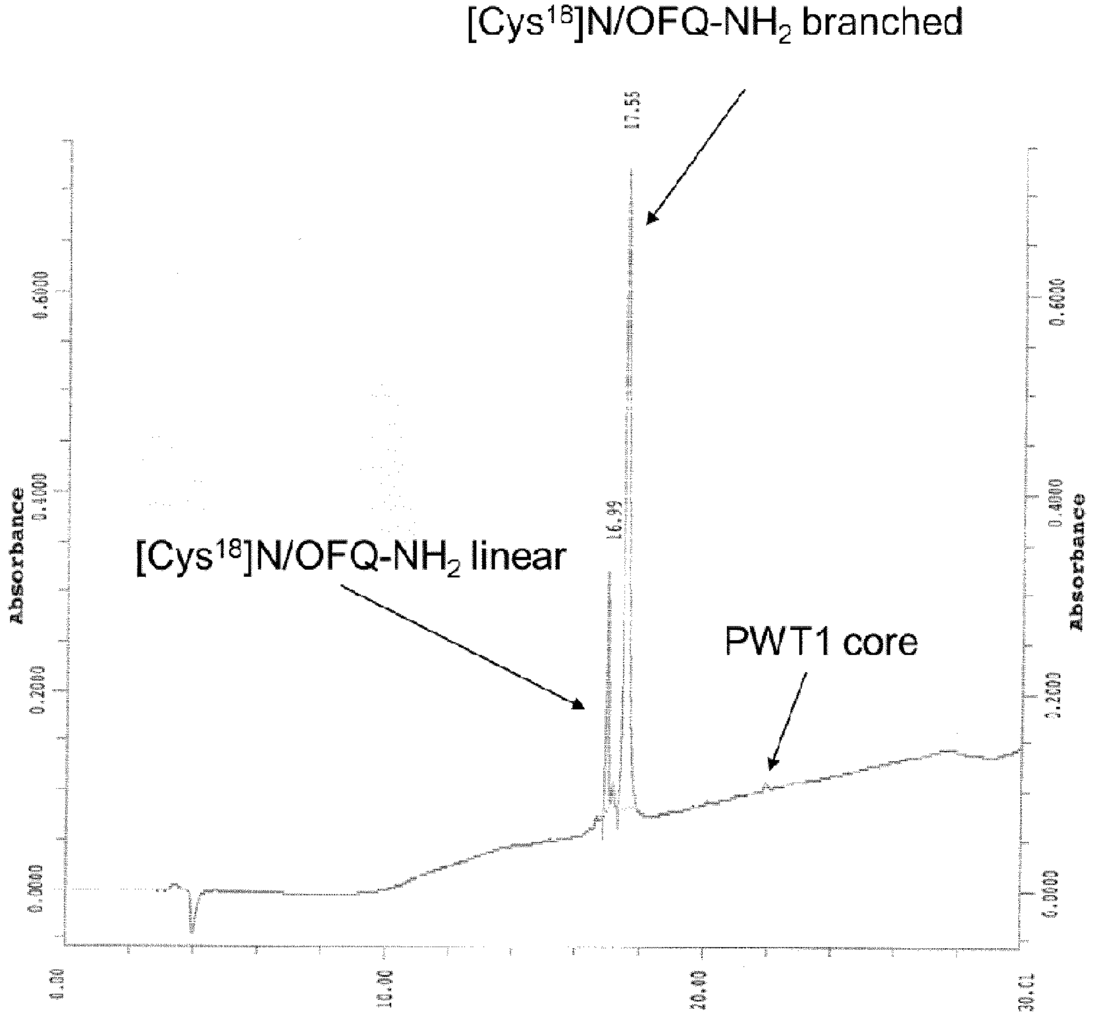

[Cys18]N/OFQ-NH2 branched
[Cys18]N/OFQ-NH2 linear
PWT1 core
17.55
16.99
Absorbance
0.6000
0.4000
0.2000
0.0000
0.00
10.00
20.00
30.01

Figure 8

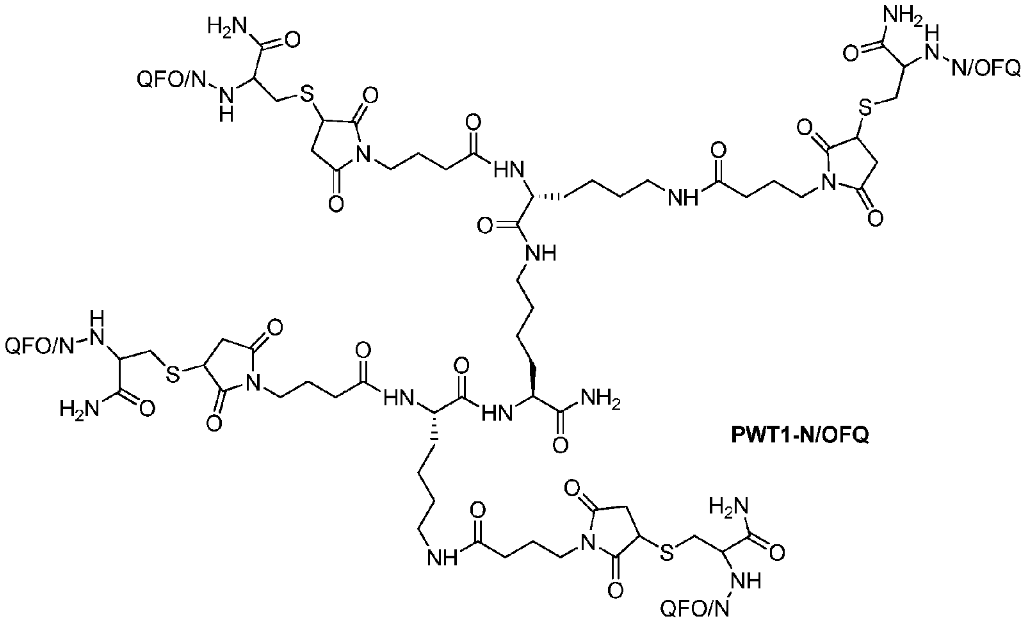

PWT1-N/OFQ

N/OFQ = Phe[1]-Gly-Gly-Phe-Thr-Gly-Ala-Arg[8]-Lys-Ser-Ala-Arg-Lys-Leu-Ala-Asn-Gln[17]

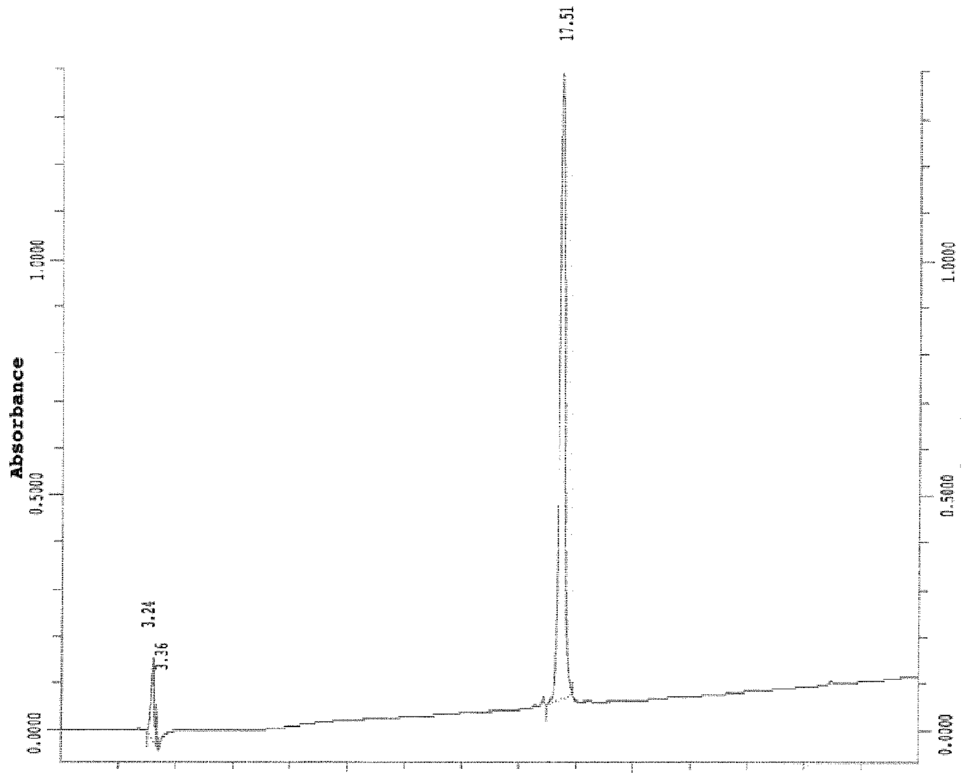

Figure 9

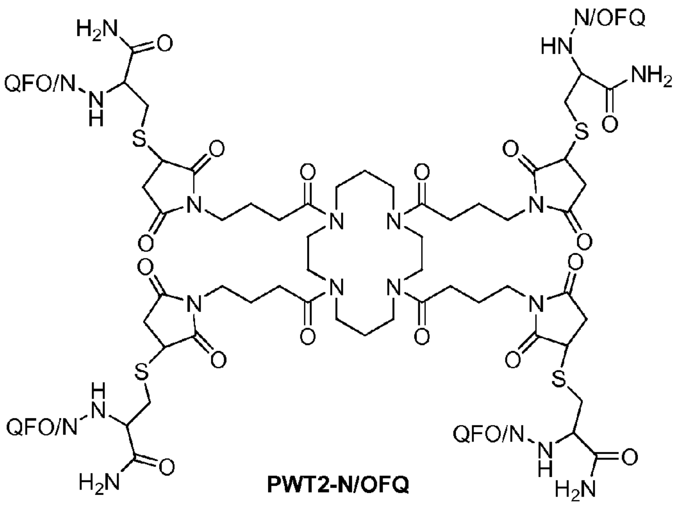

PWT2-N/OFQ

N/OFQ = Phe[1]-Gly-Gly-Phe-Thr-Gly-Ala-Arg[8]-Lys-Ser-Ala-Arg-Lys-Leu-Ala-Asn-Gln[17]

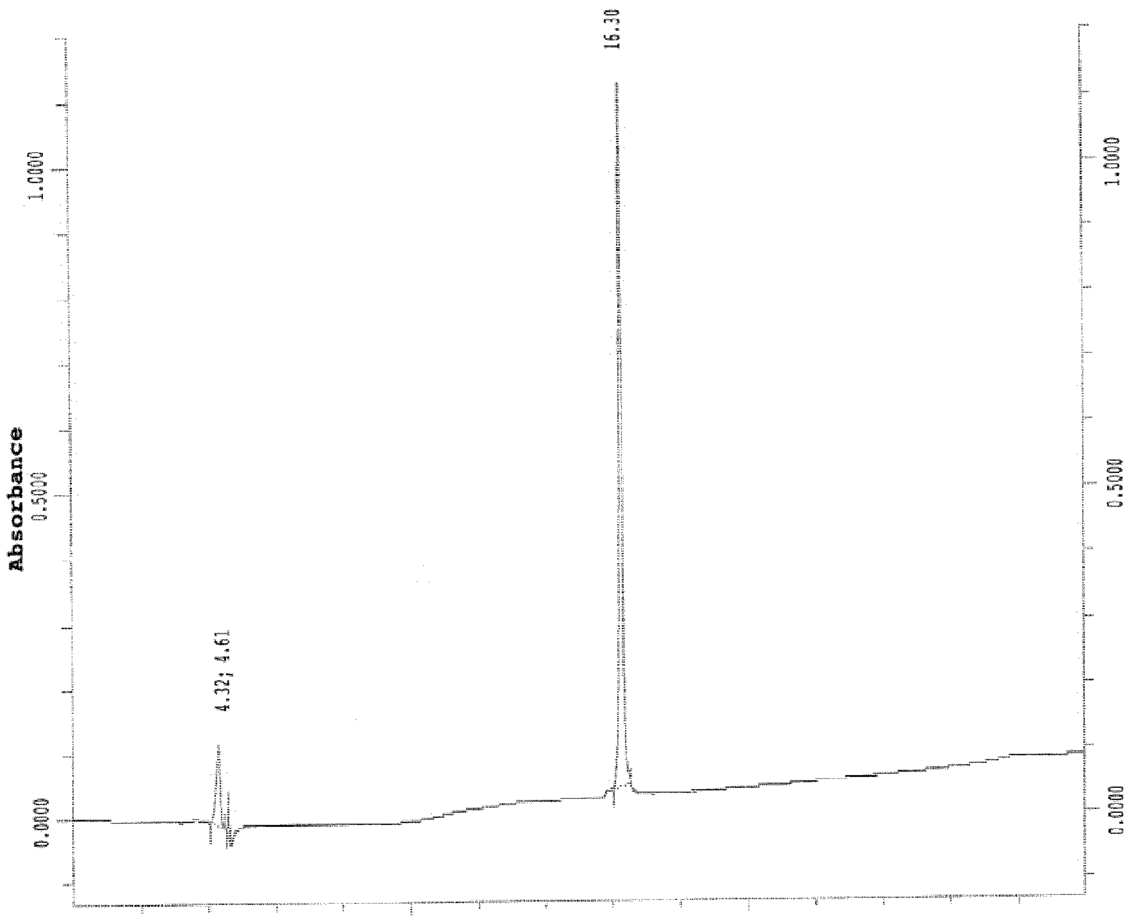

Figure 10

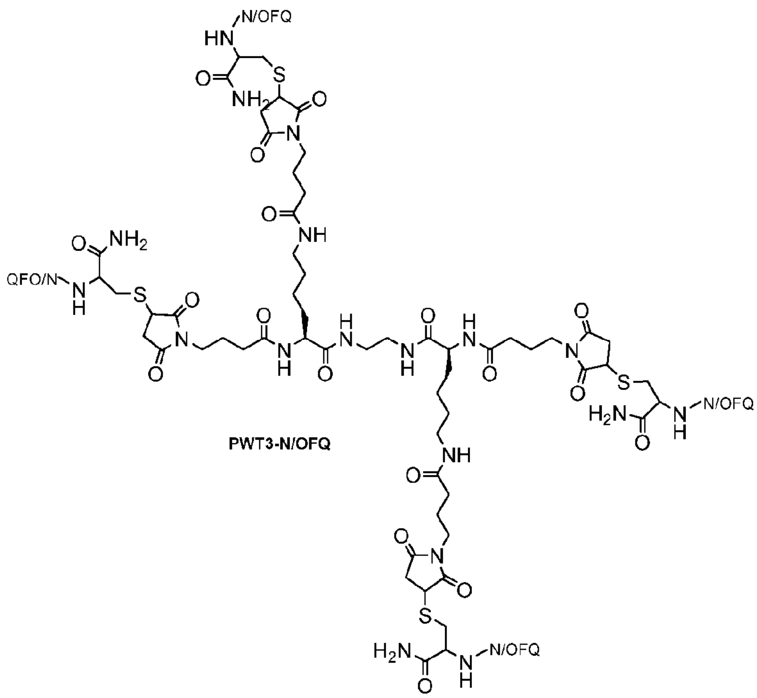

PWT3-N/OFQ

N/OFQ = Phe[1]-Gly-Gly-Phe-Thr-Gly-Ala-Arg[8]-Lys-Ser-Ala-Arg-Lys-Leu-Ala-Asn-Gln[17]

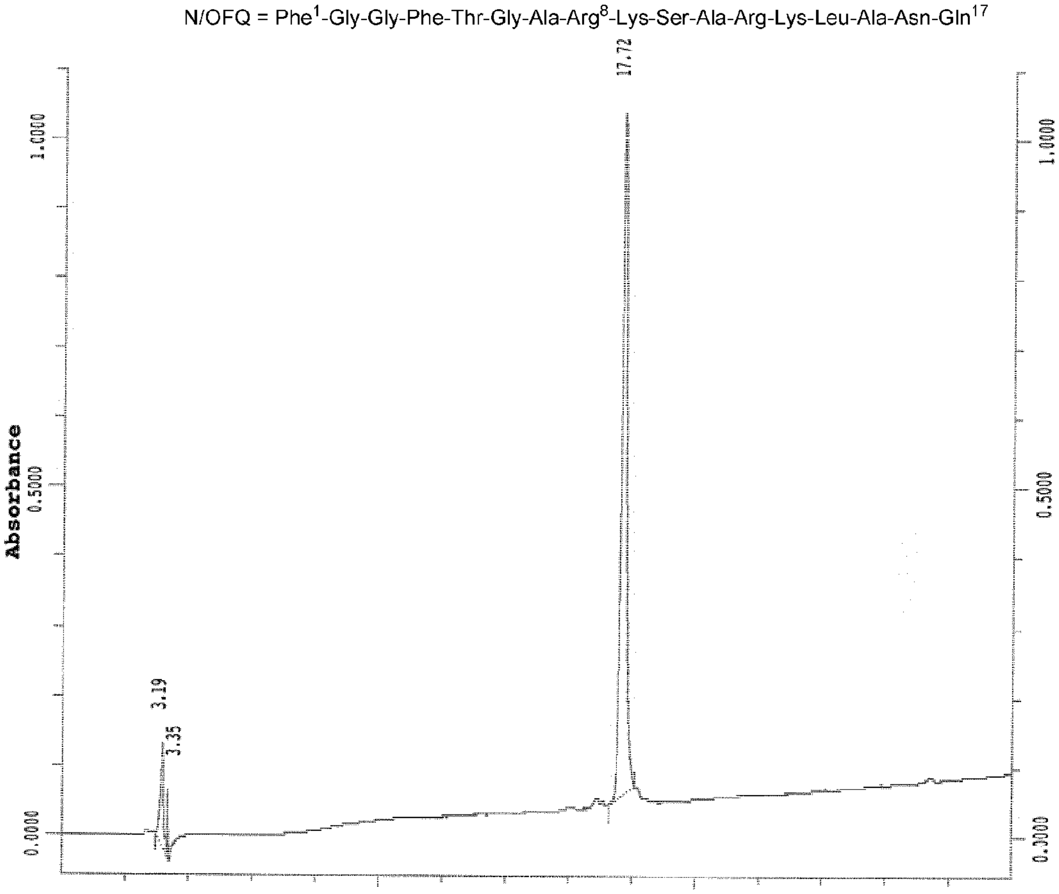

Figure 11

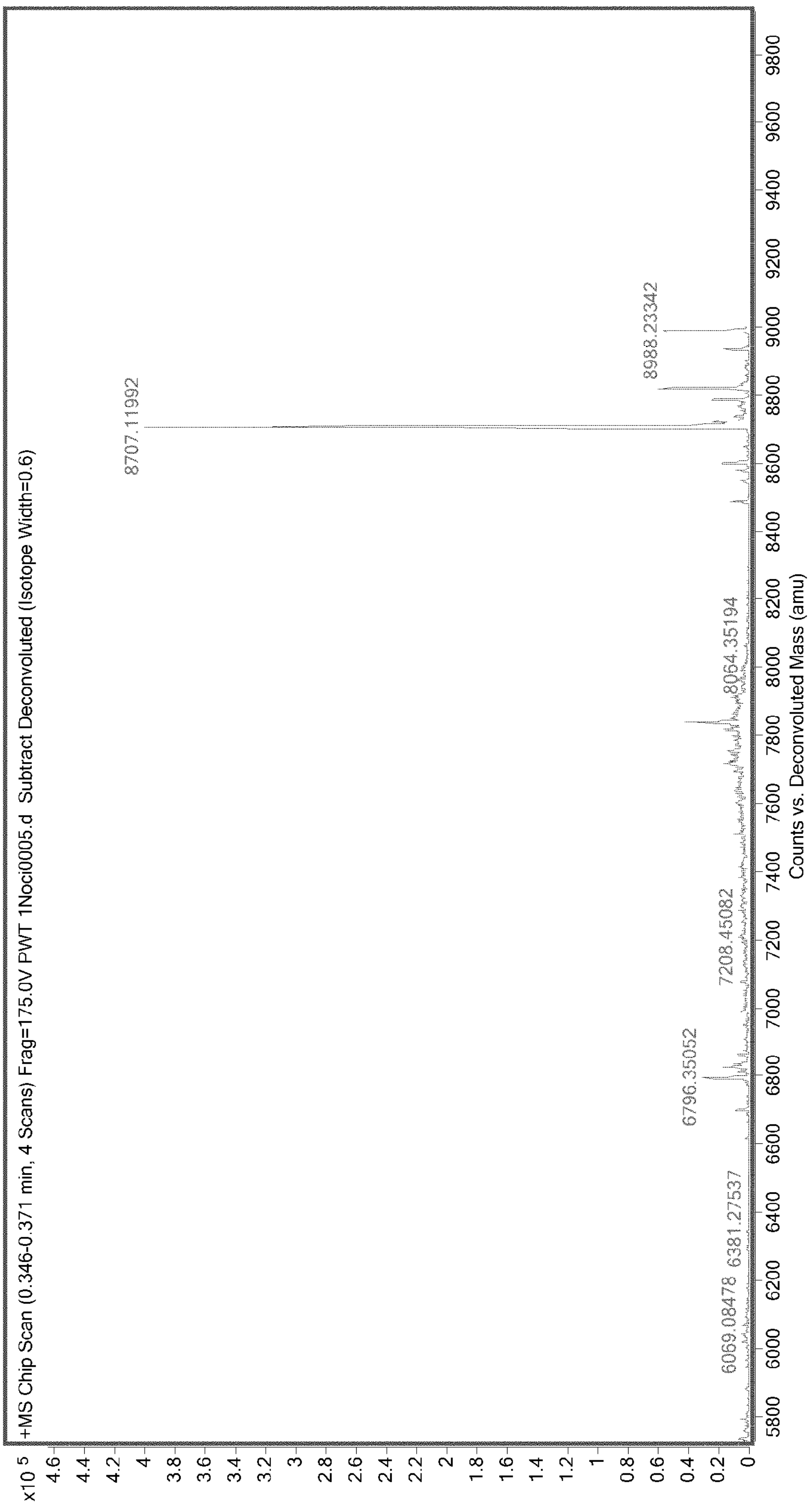
+MS Chip Scan (0.346-0.371 min, 4 Scans) Frag=175.0V PWT 1Noci0005.d Subtract Deconvoluted (Isotope Width=0.6)
x10 5
4.6
4.4
4.2
4
3.8
3.6
3.4
3.2
3
2.8
2.6
2.4
2.2
2
1.8
1.6
1.4
1.2
1
0.8
0.6
0.4
0.2
0
8707.11992
8988.23342
8064.35194
7208.45082
6796.35052
6381.27537
6069.08478
5800
6000
6200
6400
6600
6800
7000
7200
7400
7600
7800
8000
8200
8400
8600
8800
9000
9200
9400
9600
9800
Counts vs. Deconvoluted Mass (amu)

Figure 12

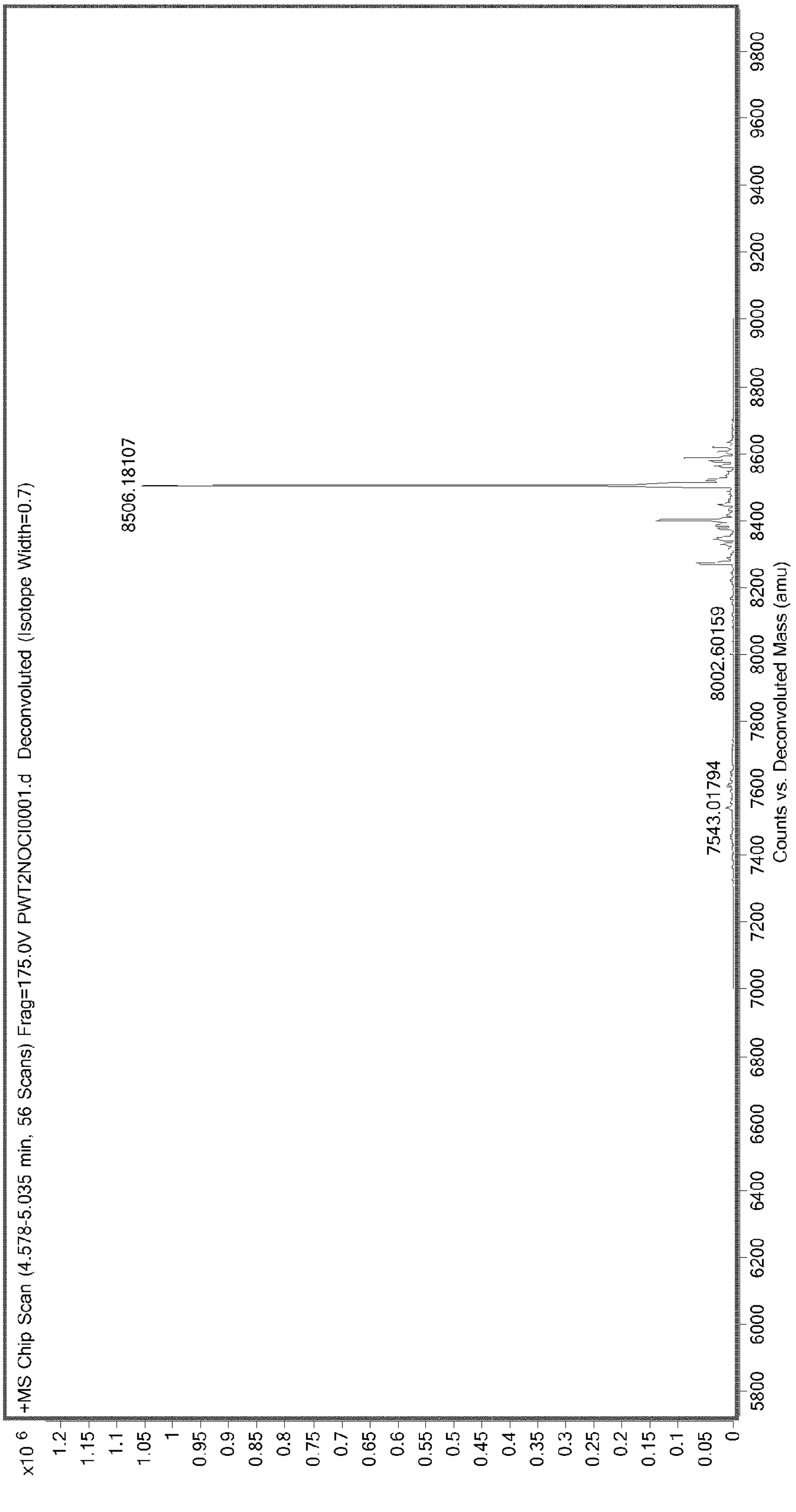
+MS Chip Scan (4.578-5.035 min, 56 Scans) Frag=175.0V PWT2NOCI0001.d Deconvoluted (Isotope Width=0.7)
x10 6
8506.18107
7543.01794
8002.60159
Counts vs. Deconvoluted Mass (amu)

Figure 13

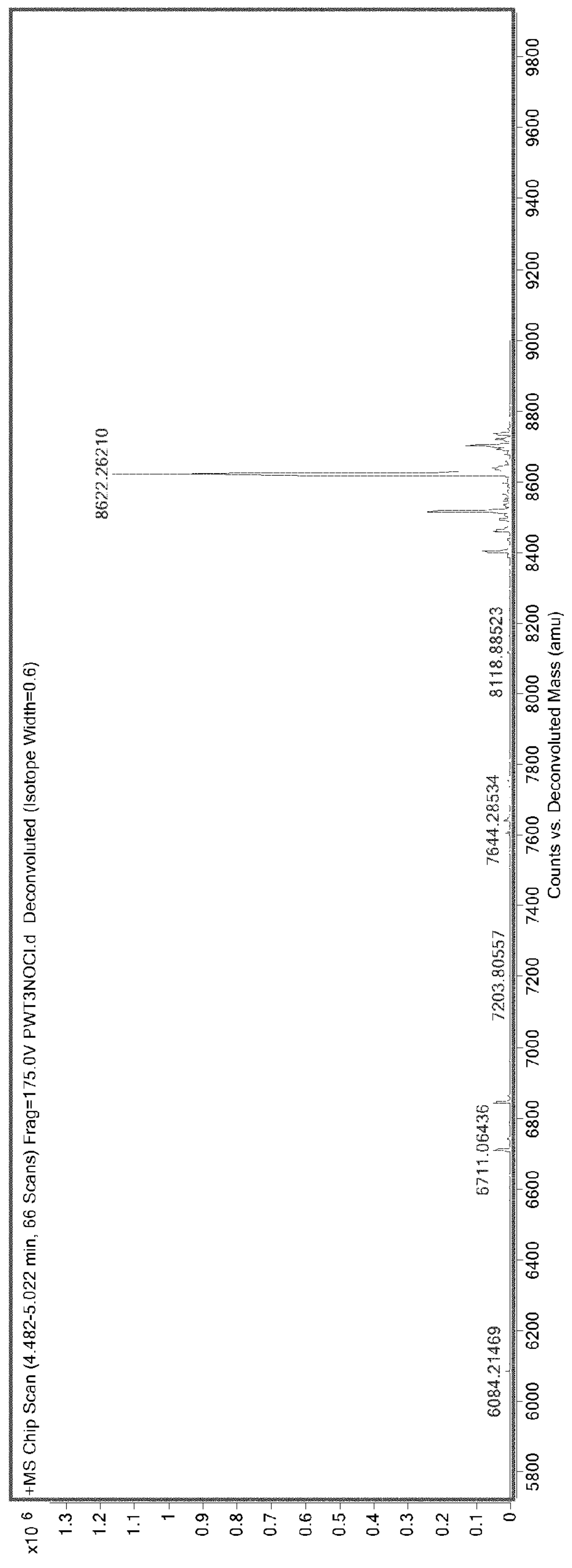
+MS Chip Scan (4.482-5.022 min, 66 Scans) Frag=175.0V PWT3NOCl.d Deconvoluted (Isotope Width=0.6)
x10 6
1.3
1.2
1.1
1
0.9
0.8
0.7
0.6
0.5
0.4
0.3
0.2
0.1
0
8622.26210
6084.21469
6711.06436
7203.80557
7644.28534
8118.88523
5800
6000
6200
6400
6600
6800
7000
7200
7400
7600
7800
8000
8200
8400
8600
8800
9000
9200
9400
9600
9800
Counts vs. Deconvoluted Mass (amu)

Figure 14

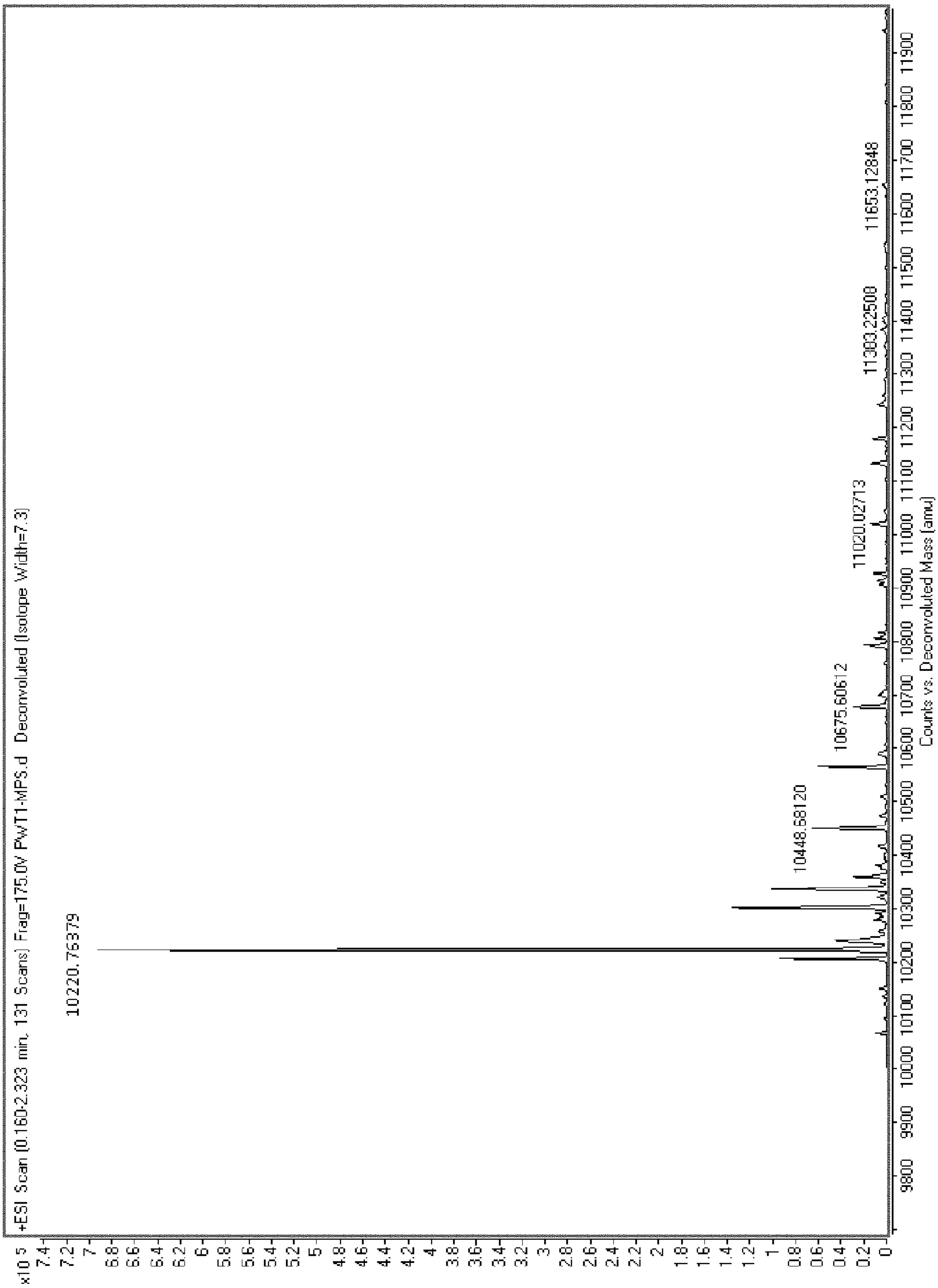
+ESI Scan (0.160-2.323 min, 131 Scans) Frag=175.0V PWT1-MPS.d  Deconvoluted (Isotope Width=7.3)
x10 5
10220.76379
10448.58120
10675.60612
11020.02713
11383.22500
11653.12848
Counts vs. Deconvoluted Mass (amu)

Figure 15

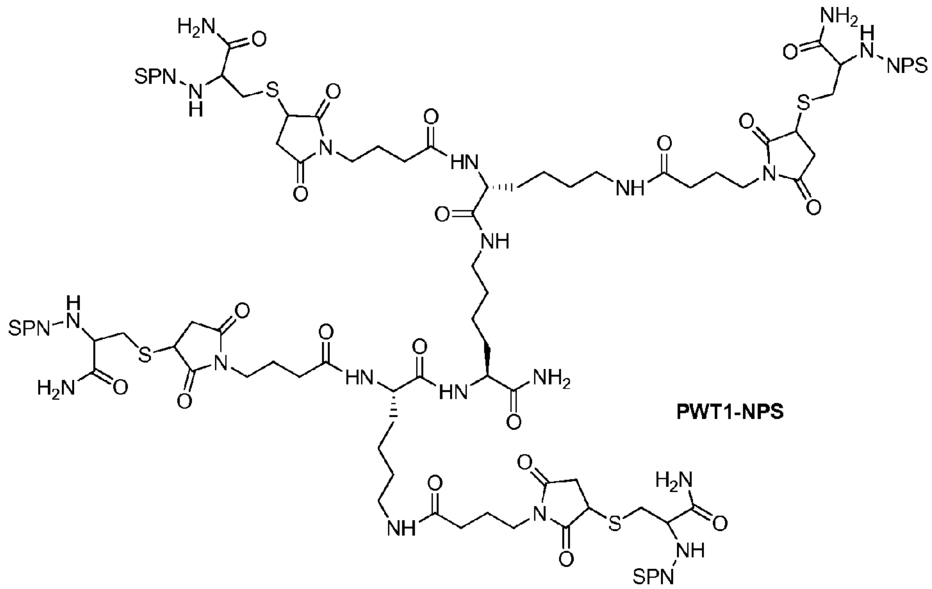

**PWT1-NPS**

NPS = Ser[1]-Phe-Arg-Asn-Gly-Val-Gly-Thr-Gly-Met[10]-Lys-Lys-Thr-Ser-Phe-Gln-Arg-Ala-Lys-Ser[20]

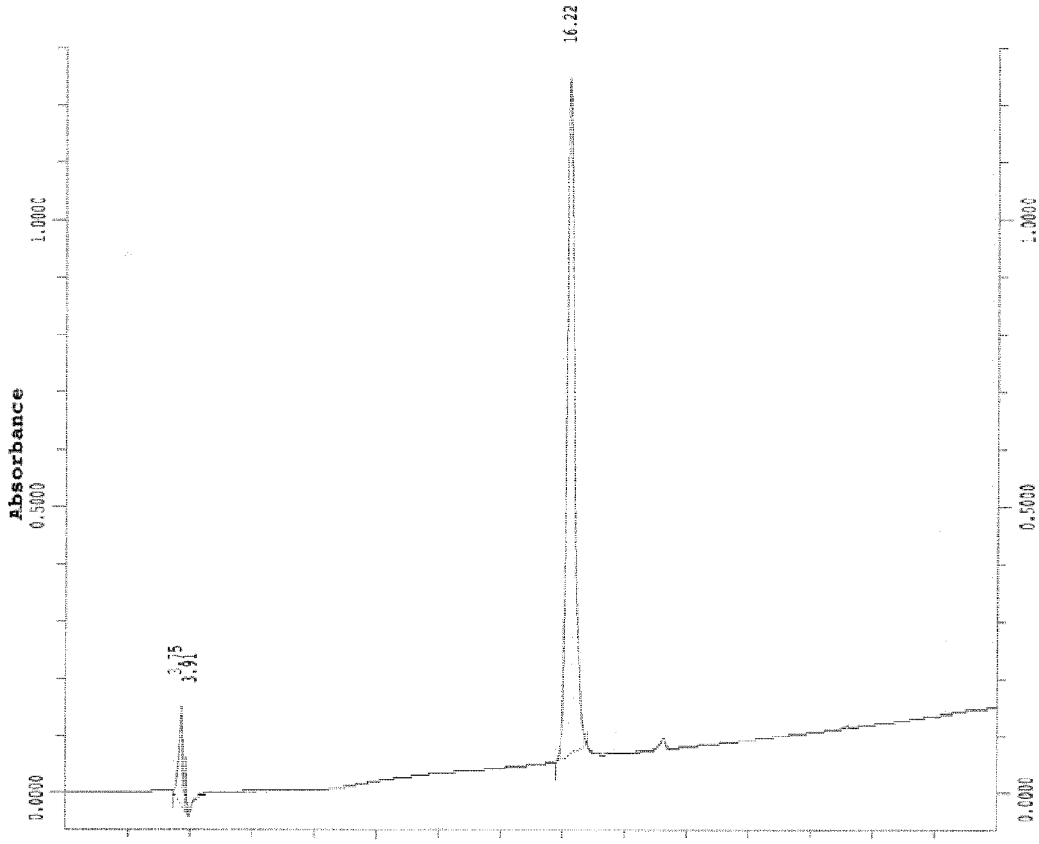

Figure 16

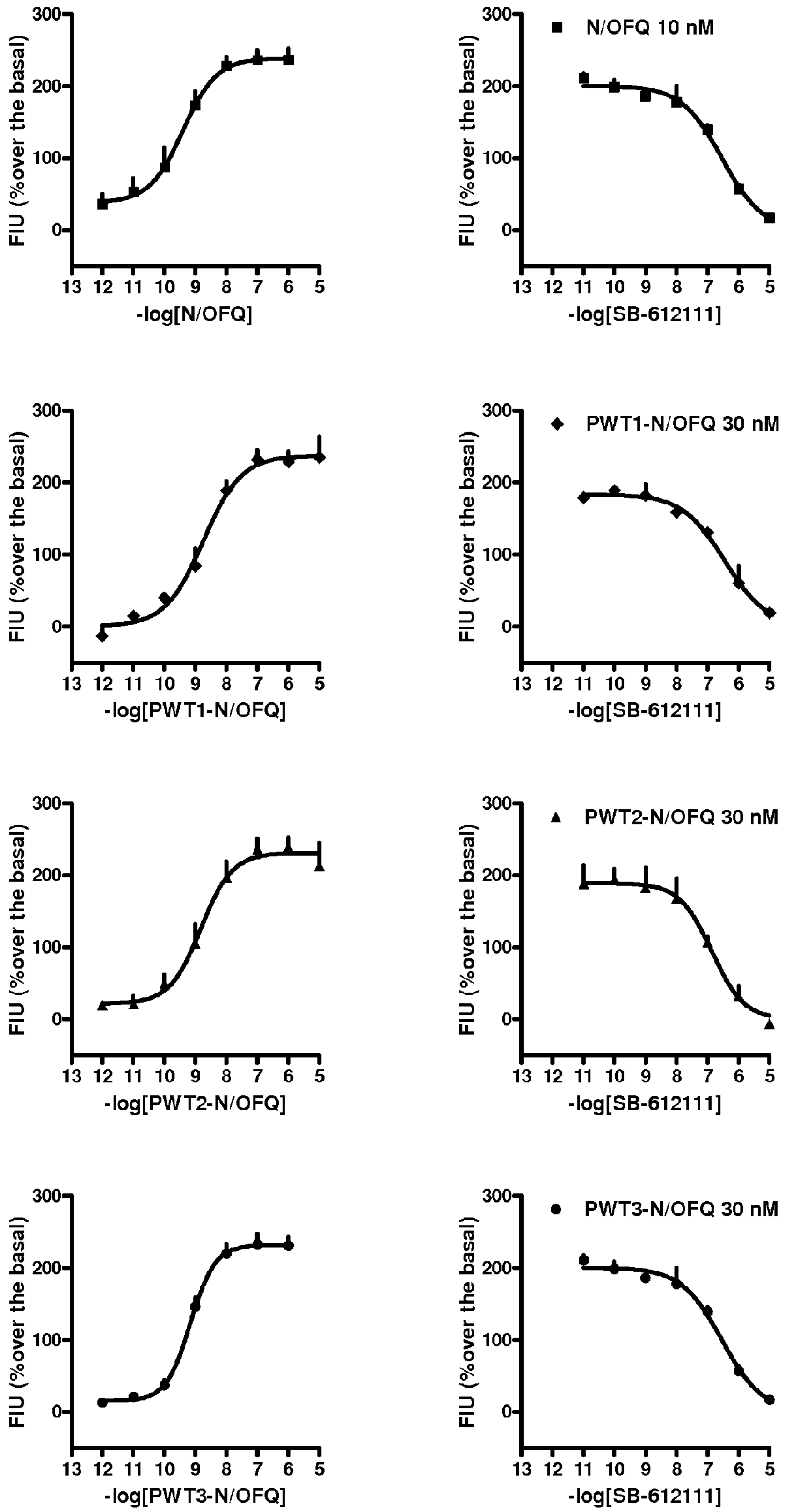
N/OFQ 10 nM
PWT1-N/OFQ 30 nM
PWT2-N/OFQ 30 nM
PWT3-N/OFQ 30 nM
FIU (%over the basal)
300
200
100
0
13 12 11 10 9 8 7 6 5
-log[N/OFQ]
-log[PWT1-N/OFQ]
-log[PWT2-N/OFQ]
-log[PWT3-N/OFQ]
-log[SB-612111]

Figure 17

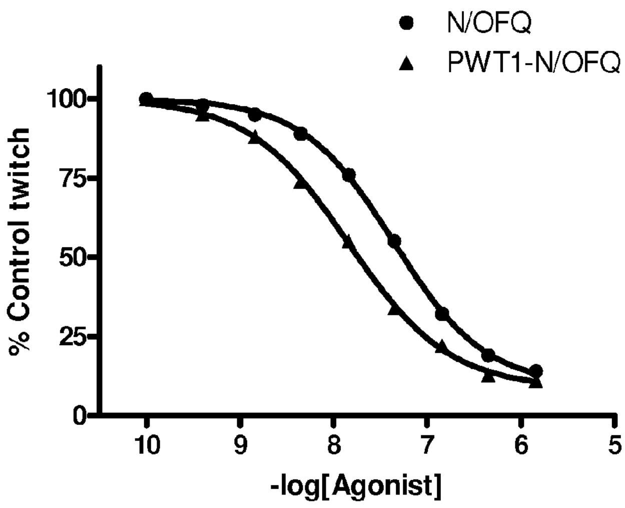
N/OFQ
PWT1-N/OFQ
% Control twitch
100
75
50
25
0
10
9
8
7
6
5
-log[Agonist]

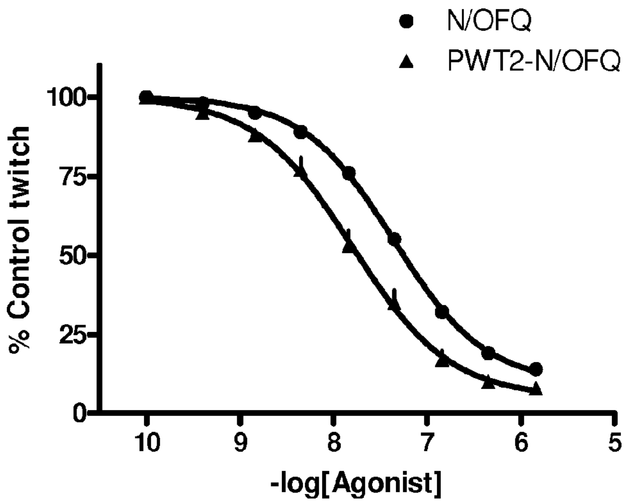
N/OFQ
PWT2-N/OFQ
% Control twitch
100
75
50
25
0
10
9
8
7
6
5
-log[Agonist]

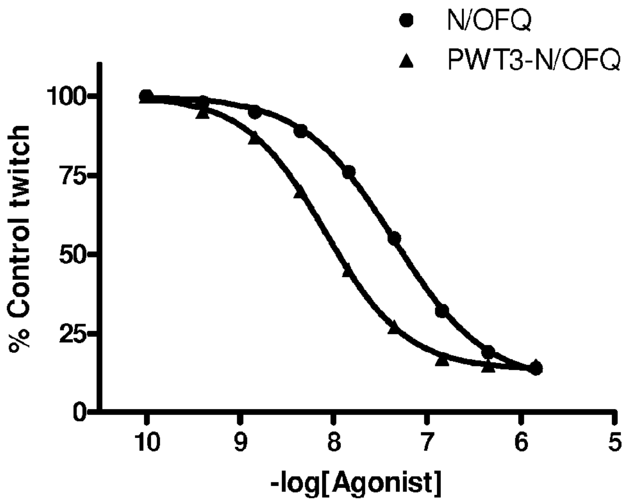
N/OFQ
PWT3-N/OFQ
% Control twitch
100
75
50
25
0
10
9
8
7
6
5
-log[Agonist]

Figure 18

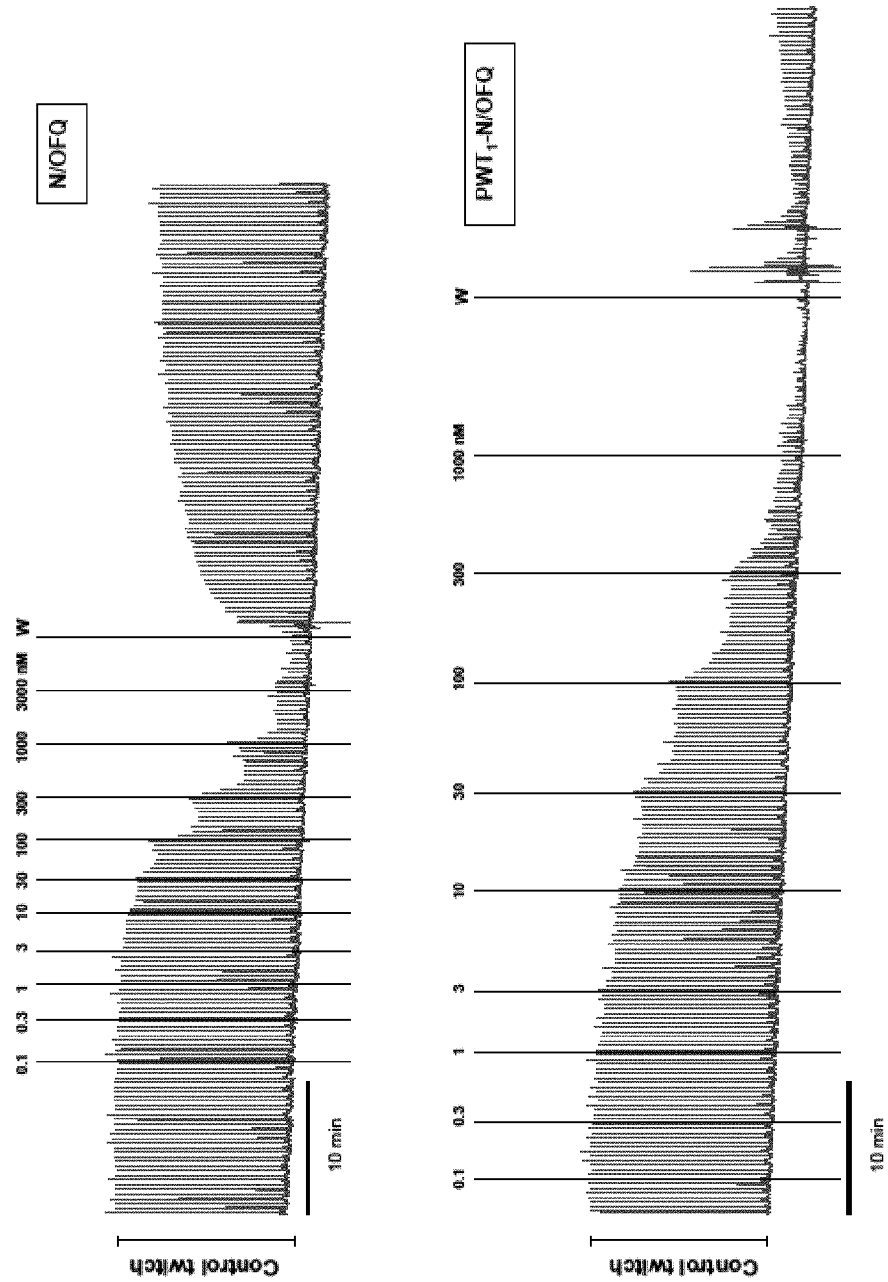
N/OFQ
0.1
0.3
1
3
10
30
100
300
1000
3000 nM
W
10 min
Control twitch
PWT$_1$-N/OFQ
0.1
0.3
1
3
10
30
100
300
1000 nM
W
10 min
Control twitch

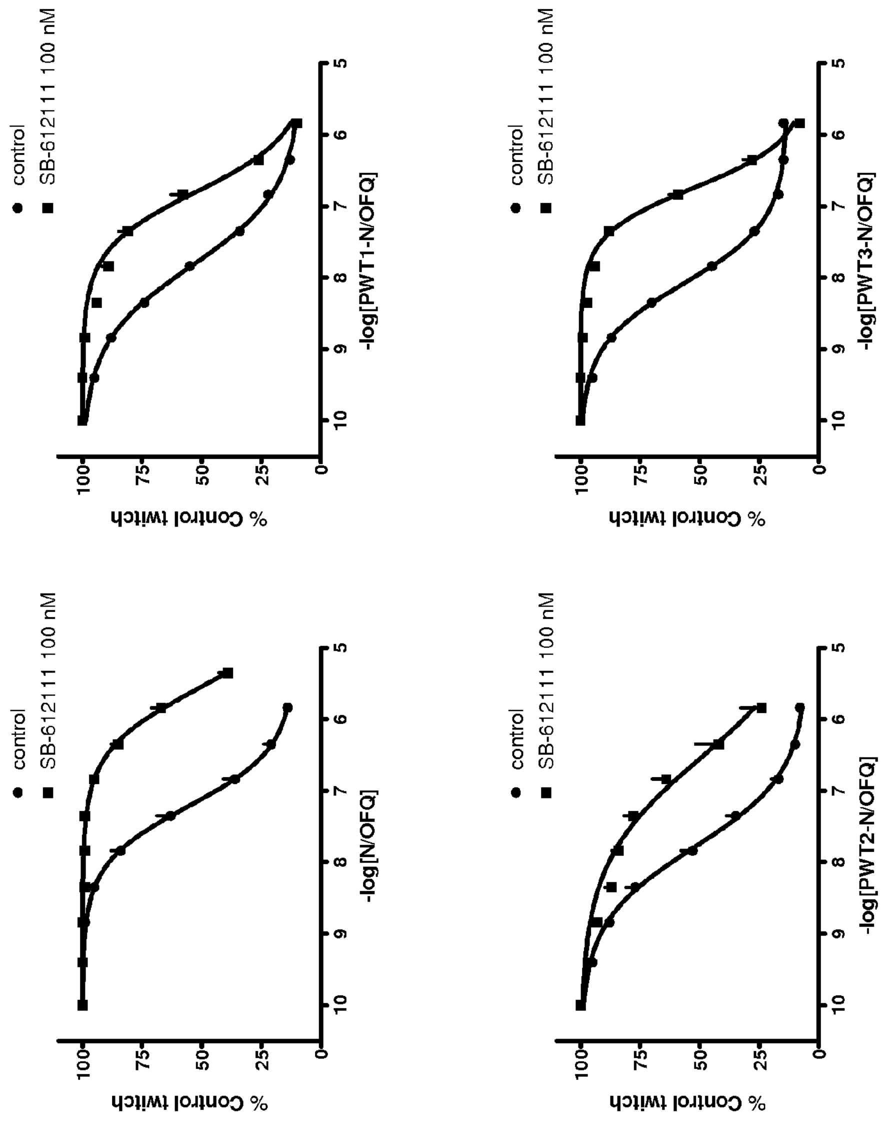
control
SB-612111 100 nM
% Control twitch
-log[N/OFQ]
-log[PWT1-N/OFQ]
-log[PWT2-N/OFQ]
-log[PWT3-N/OFQ]
0
25
50
75
100
10
9
8
7
6
5

Figure 19

Figure 20

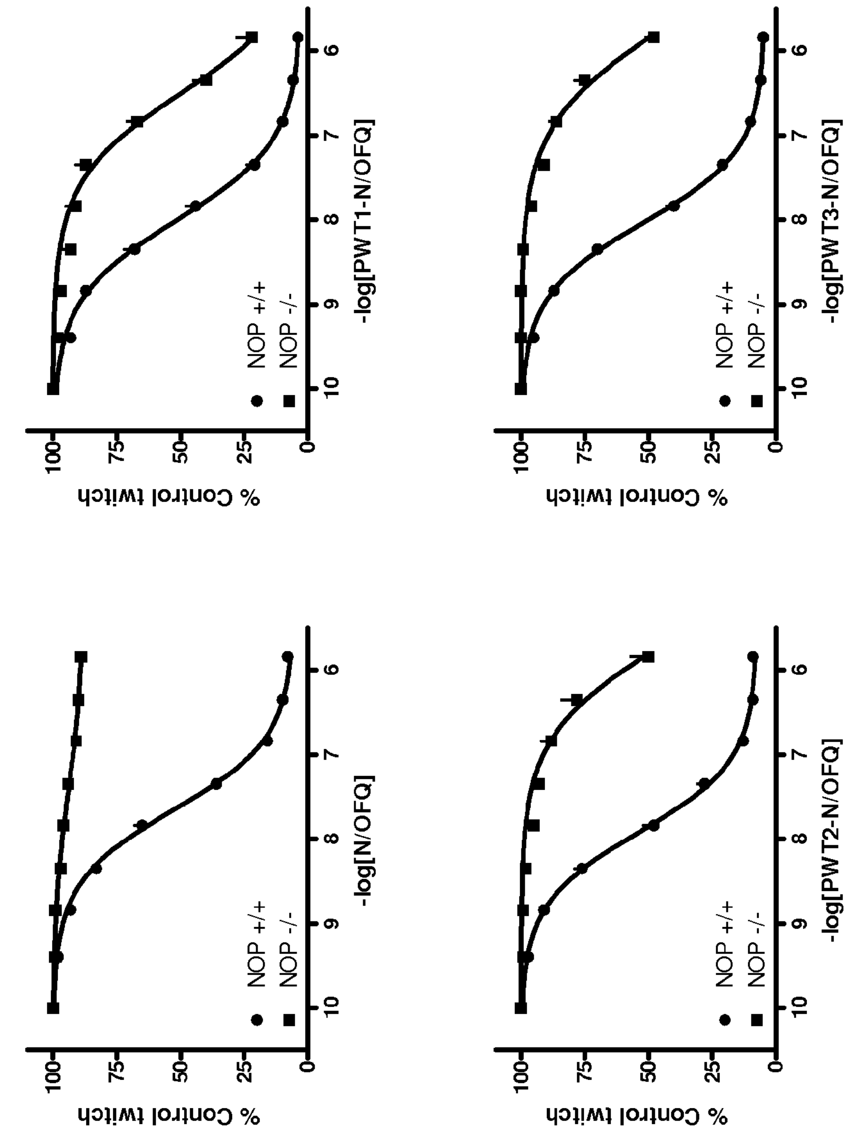
% Control twitch
-log[N/OFQ]
-log[PWT1-N/OFQ]
-log[PWT2-N/OFQ]
-log[PWT3-N/OFQ]
NOP +/+
NOP -/-

Figure 21

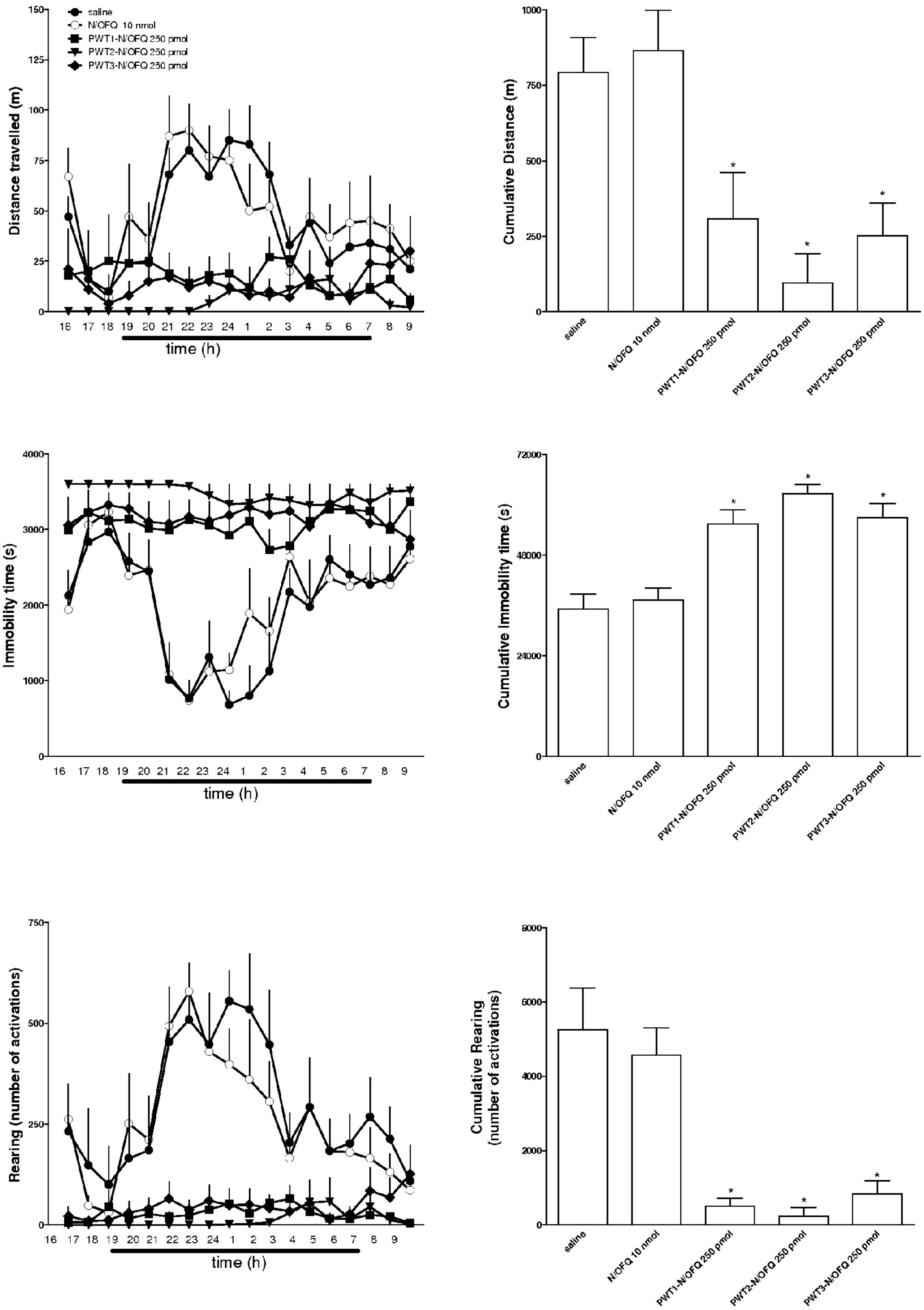
saline
N/OFQ 10 nmol
PWT1-N/OFQ 250 pmol
PWT2-N/OFQ 250 pmol
PWT3-N/OFQ 250 pmol
Distance travelled (m)
time (h)
Cumulative Distance (m)
Immobility time (s)
Cumulative Immobility time (s)
Rearing (number of activations)
Cumulative Rearing (number of activations)
saline
N/OFQ 10 nmol
PWT1-N/OFQ 250 pmol
PWT2-N/OFQ 250 pmol
PWT3-N/OFQ 250 pmol

Figure 22

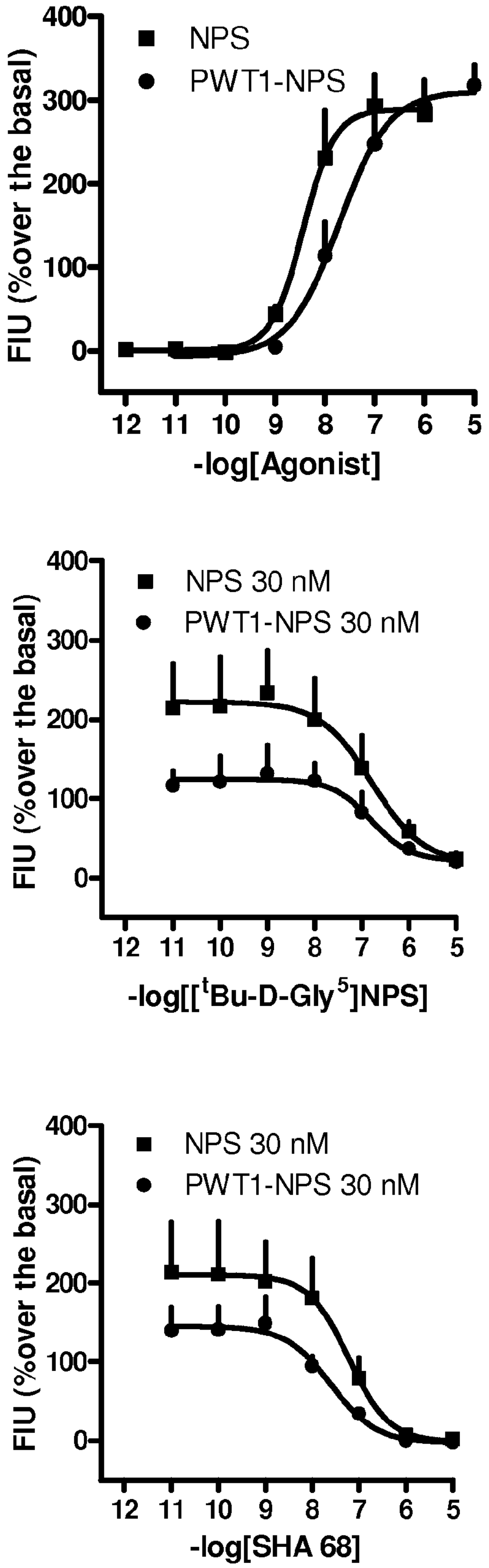
400
300
200
100
0
FIU (%over the basal)
NPS
PWT1-NPS
12 11 10 9 8 7 6 5
-log[Agonist]
NPS 30 nM
PWT1-NPS 30 nM
-log[[tBu-D-Gly5]NPS]
NPS 30 nM
PWT1-NPS 30 nM
-log[SHA 68]

Europäisches Patentamt
European Patent Office
Office européen des brevets

# EUROPEAN SEARCH REPORT

Application Number
EP 13 16 2532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 92/22583 A2 (CELLTECH LTD [GB]) 23 December 1992 (1992-12-23)<br>* page 7, lines 6-13 *<br>* page 36, lines 10-26 *<br>* page 41, line 15 - page 43, line 12 *<br>----- | 1-3,5,8, 10-15<br>1,2,4,6, 7,9,11, 14,15 | INV.<br>A61K47/48<br><br>ADD.<br>C07K7/06<br>C07K7/08<br>A61K38/08<br>A61K38/10 |
| X<br><br>Y | LI, SHUNZI ET AL: "Synthesis and characterization of a high-affinity .alpha.v.beta.6-specific ligand for in vitro and in vivo applications", MOLECULAR CANCER THERAPEUTICS , 8(5), 1239-1249 CODEN: MCTOCF; ISSN: 1535-7163, 2009, XP002711908,<br>* page 1239, right-hand column, paragraph 3 - page 1240, left-hand column, paragraph 1 *<br>* figures 1,5 *<br>----- | 1-3,5,8, 10-15<br><br>1,2,4,6, 7,9,11, 14,15 | |
| X<br><br>Y | SHUNZI LI ET AL: "Synthesis and biological evaluation of a peptidepaclitaxel conjugate which targets the integrin", BIOORGANIC & MEDICINAL CHEMISTRY, PERGAMON, GB, vol. 19, no. 18, 22 July 2011 (2011-07-22) , pages 5480-5489, XP028389413, ISSN: 0968-0896, DOI: 10.1016/J.BMC.2011.07.046 [retrieved on 2011-08-04]<br>* abstract *<br>* scheme 2 *<br>* figure 1 *<br>* page 5847, sections 4.4 and 4.5 *<br>-----<br>-/-- | 1-3,5,8, 10-15<br><br>1,2,4,6, 7,9,11, 14,15 | TECHNICAL FIELDS SEARCHED (IPC)<br><br>A61K<br>C07K |

The present search report has been drawn up for all claims

| Place of search | Date of completion of the search | Examiner |
|---|---|---|
| The Hague | 29 October 2013 | Villard, Anne-Laure |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 2532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2007/144685 A1 (COMMISSARIAT ENERGIE ATOMIQUE [FR]; SAUVAGE VITA MIREILLE [FR]; VITA F) 21 December 2007 (2007-12-21)<br>* page 7, lines 23-29 *<br>* page 15, lines 28-32 *<br>* example 3; page 37, lines 22, 27; page 38, lines 20-26 *<br>* figure 15 *<br>----- | 1-3,5,8, 10-15<br><br>1,2,4,6, 7,9,11, 14,15 | |
| Y | BRACCI LUISA ET AL: "Synthetic peptides in the form of dendrimers become resistant to protease activity",<br>JOURNAL OF BIOLOGICAL CHEMISTRY, AMERICAN SOCIETY FOR BIOCHEMISTRY AND MOLECULAR BIOLOGY,<br>vol. 278, no. 47,<br>21 November 2003 (2003-11-21), pages 46590-46595, XP002461778,<br>ISSN: 0021-9258, DOI: 10.1074/JBC.M308615200<br>* the whole document *<br>----- | 6,7 | TECHNICAL FIELDS SEARCHED (IPC) |
| A | US 2005/159341 A1 (WANG LAI-XI [US] ET AL) 21 July 2005 (2005-07-21)<br>* paragraphs [0006], [0035] *<br>* figure 2; compounds 8,10 *<br>* figures 3,6,7 *<br>----- | 1,11,14, 15 | |
| A | US 2008/241102 A1 (HERSEL ULRICH [DE] ET AL) 2 October 2008 (2008-10-02)<br>* page 38 *<br>----- | 1,11,14, 15 | |
| A | WO 2012/030683 A2 (MERCK SHARP & DOHME [US]; COLLETTI STEVEN L [US]; GOSSELIN FRANCIS [US) 8 March 2012 (2012-03-08)<br>* page 22, last paragraph - page 23 *<br>-----<br>-/-- | 1,11,14, 15 | |

The present search report has been drawn up for all claims

3

| Place of search | Date of completion of the search | Examiner |
|---|---|---|
| The Hague | 29 October 2013 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 2532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2009/082537 A1 (RAMON HERNANDEZ JOSE ANGEL [CU] ET AL) 26 March 2009 (2009-03-26) * paragraphs [0165] - [0191], [0207] - [0218] * * figure 4 * ----- | 1,11,14, 15 | |
| Y | RÖHRICH ANIKA ET AL: "A novel tetrabranched neurotensin(8-13) cyclam derivative: synthesis, 64Cu-labeling and biological evaluation.", JOURNAL OF INORGANIC BIOCHEMISTRY JUN 2011, vol. 105, no. 6, June 2011 (2011-06), pages 821-832, XP002715530, ISSN: 1873-3344 * Scheme 2 * * page 831, section "Conclusion" * ----- | 1,2,4,6, 7,9,11, 14,15 | |
| Y | ZHANG XIAOFEN ET AL: "Macrocyclic chelator assembled RGD multimers for tumor targeting", BIOORGANIC & MEDICINAL CHEMISTRY LETTERS, vol. 21, no. 11, June 2011 (2011-06), pages 3423-3426, XP002715531, * abstract * * figure 1 * ----- -/-- | 1,2,4,9, 11,14,15 | TECHNICAL FIELDS SEARCHED (IPC) |

The present search report has been drawn up for all claims

| Place of search | Date of completion of the search | Examiner |
|---|---|---|
| The Hague | 29 October 2013 | Villard, Anne-Laure |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 2532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KRAUS J-L ET AL: "Cyclic tetrameric clusters of chemotactic peptides as superactive activators of lysozyme release from human neutrophils", BIOCHEMICAL AND BIOPHYSICAL RESEARCH COMMUNICATIONS, ACADEMIC PRESS INC. ORLANDO, FL, US, vol. 124, no. 3, 14 November 1984 (1984-11-14), pages 939-944, XP024841396, ISSN: 0006-291X, DOI: 10.1016/0006-291X(84)91048-9 [retrieved on 1984-11-14] * page 942; table 1 * ----- | 1,2,4,9, 11,14,15 | |
| A | LI ET AL: "Synthesis and anti-HIV activity of trivalent CD4-mimetic miniproteins", BIOORGANIC & MEDICINAL CHEMISTRY, PERGAMON, GB, vol. 15, no. 12, 5 May 2007 (2007-05-05), pages 4220-4228, XP022062555, ISSN: 0968-0896, DOI: 10.1016/J.BMC.2007.03.064 * abstract * * Schemes 1 and 3 * * pages 4224-4225, section "Conclusion" * ----- | 1,2,4,6, 7,9,11, 14,15 | TECHNICAL FIELDS SEARCHED (IPC) |

The present search report has been drawn up for all claims

3

| Place of search | Date of completion of the search | Examiner |
|---|---|---|
| The Hague | 29 October 2013 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 13 16 2532

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 13 16 2532

The Search Division considers that the present European patent application does not comply with the requirements of unity of invention and relates to several inventions or groups of inventions, namely:

1. claims: 3, 5, 8, 10(completely); 1, 2, 6, 7, 11-15(partially)

A supramolecular aggregate of formula (I) (as referred to in claim 1) with X being a group consisting of formulae (III) or (V) (as referred to in claim 2);
The macromolecular cores PTW1 and PTW3 as referred to in claims 8 and 10 ;
A process for preparing the aforementioned aggregate;
Said aggregate for use in targeting the attached active substance to the target site.

---

2. claims: 4, 9(completely); 1, 2, 6, 7, 11-15(partially)

A supramolecular aggregate of formula (I) (as referred to in claim 1) with X being a group consisting of formula (IV) (as referred to in claim 2);
The macromolecular cores PTW2 as referred to in claim 9;
A process for preparing the aforementioned aggregate;
Said aggregate for use in targeting the attached active substance to the target site.

---

## ANNEX TO THE EUROPEAN SEARCH REPORT ON EUROPEAN PATENT APPLICATION NO. EP 13 16 2532

This annex lists the patent family members relating to the patent documents cited in the above-mentioned European search report. The members are as contained in the European Patent Office EDP file on
The European Patent Office is in no way liable for these particulars which are merely given for the purpose of information.

29-10-2013

| Patent document cited in search report | Publication date | Patent family member(s) | Publication date |
|---|---|---|---|
| WO 9222583 A2 | 23-12-1992 | AT 192457 T | 15-05-2000 |
| | | AU 1971692 A | 12-01-1993 |
| | | CA 2088367 A1 | 12-12-1992 |
| | | DE 69230994 D1 | 08-06-2000 |
| | | DE 69230994 T2 | 01-02-2001 |
| | | DK 0560947 T3 | 07-08-2000 |
| | | EP 0560947 A1 | 22-09-1993 |
| | | ES 2146212 T3 | 01-08-2000 |
| | | FI 930580 A | 02-04-1993 |
| | | JP 3373849 B2 | 04-02-2003 |
| | | JP H06502657 A | 24-03-1994 |
| | | NO 930440 A | 02-04-1993 |
| | | NZ 243099 A | 26-07-1996 |
| | | WO 9222583 A2 | 23-12-1992 |
| | | ZA 9204271 A | 13-12-1993 |
| WO 2007144685 A1 | 21-12-2007 | AT 482978 T | 15-10-2010 |
| | | CN 101522710 A | 02-09-2009 |
| | | EP 2035453 A2 | 18-03-2009 |
| | | ES 2371471 T3 | 03-01-2012 |
| | | US 2009285798 A1 | 19-11-2009 |
| | | WO 2007144685 A1 | 21-12-2007 |
| | | WO 2008010088 A2 | 24-01-2008 |
| US 2005159341 A1 | 21-07-2005 | AU 2003245648 A1 | 06-01-2004 |
| | | CA 2490124 A1 | 31-12-2003 |
| | | EP 1532159 A2 | 25-05-2005 |
| | | US 2005159341 A1 | 21-07-2005 |
| | | WO 2004000802 A2 | 31-12-2003 |
| US 2008241102 A1 | 02-10-2008 | AU 2005232371 A1 | 27-10-2005 |
| | | AU 2010212335 A1 | 09-09-2010 |
| | | BR PI0507875 A | 24-07-2007 |
| | | CA 2602705 A1 | 27-10-2005 |
| | | CA 2786794 A1 | 27-10-2005 |
| | | CA 2824093 A1 | 27-10-2005 |
| | | EP 1732607 A2 | 20-12-2006 |
| | | EP 2087910 A2 | 12-08-2009 |
| | | JP 4950022 B2 | 13-06-2012 |
| | | JP 2007530485 A | 01-11-2007 |
| | | JP 2012072142 A | 12-04-2012 |
| | | US 2008241102 A1 | 02-10-2008 |
| | | US 2013150281 A1 | 13-06-2013 |
| | | WO 2005099768 A2 | 27-10-2005 |
| WO 2012030683 A2 | 08-03-2012 | AU 2011296268 A1 | 21-02-2013 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 16 2532

This annex lists the patent family members relating to the patent documents cited in the above-mentioned European search report. The members are as contained in the European Patent Office EDP file on
The European Patent Office is in no way liable for these particulars which are merely given for the purpose of information.

29-10-2013

| Patent document cited in search report | Publication date | Patent family member(s) | Publication date |
|---|---|---|---|
| | | CA 2809439 A1 | 08-03-2012 |
| | | CN 103221549 A | 24-07-2013 |
| | | EP 2611927 A1 | 10-07-2013 |
| | | US 2013253168 A1 | 26-09-2013 |
| | | WO 2012030683 A2 | 08-03-2012 |
| US 2009082537 A1 | 26-03-2009 | AR 058841 A1 | 27-02-2008 |
| | | AU 2006319636 A1 | 07-06-2007 |
| | | BR PI0604313 A | 30-01-2007 |
| | | CA 2631335 A1 | 07-06-2007 |
| | | CN 101389354 A | 18-03-2009 |
| | | CU 23556 A1 | 20-07-2010 |
| | | EP 1967212 A2 | 10-09-2008 |
| | | JP 5123201 B2 | 23-01-2013 |
| | | JP 2009517414 A | 30-04-2009 |
| | | KR 20080072960 A | 07-08-2008 |
| | | US 2009082537 A1 | 26-03-2009 |
| | | UY 29981 A1 | 29-06-2007 |
| | | WO 2007062610 A2 | 07-06-2007 |
| | | ZA 200804694 A | 25-03-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **B.D. MATHER et al.** *Prog. Polym. Sci.,* 2006, vol. 31, 487-531
- **K. SADLER et al.** *Reviews in Molecular Biotechnology,* 2002, vol. 90, 195-229
- **K. J. CHANG et al.** *Proc. Natl. Acad. Sci. USA,* 1988, vol. 85, 4929-4933
- **A. PINI et al.** *Current Protein and Peptide Science,* 2008, vol. 9, 468-477
- **L. BRACCI et al.** *J. Biol. Chem.,* 2003, vol. 278, 56590-56595
- **BENOITON, N. L.** Chemistry of Peptide Synthesis. Taylor & Francis, 2005, 125-154
- **CAMARDA et al.** *Naunyn Schmiedebergs Arch Pharmacol.,* 2009, vol. 379, 599-607
- **CAMARDA ; CALO.** *Methods Mol Biol.,* vol. 937 (2013), 293-306
- **CALO et al.** *Eur J Pharmacol.,* 1996, vol. 311, R3-5
- **GUERRINI et al.** *J Med Chem.,* 2009, vol. 52, 524-529
- **LAURSEN ; BELKNAP.** *J Pharmacol Methods,* 1986, vol. 16, 355-357
- **REISINE.** *Neuropharmacology,* 1995, vol. 34, 463-472
- **SPAGNOLO et al.** *J Pharmacol Exp Ther.,* 2007, vol. 321, 961-967
- **FISCHETTI et al.** *Peptides,* 2009, vol. 30, 248-255
- **CALO et al.** *Br J Pharmacol.,* 2000, vol. 129, 1261-1283
- **GUERRINI et al.** *Med Res Rev.,* 2010, vol. 30, 751-777